(19) Europäisches Patentamt
European Patent Office
Office européen des brevets

(11) EP 2 791 125 B1

(12) EUROPÄISCHE PATENTSCHRIFT

(45) Veröffentlichungstag und Bekanntmachung des Hinweises auf die Patenterteilung:
**23.08.2017 Patentblatt 2017/34**

(21) Anmeldenummer: **12799184.2**

(22) Anmeldetag: **11.12.2012**

(51) Int Cl.:
***C07D 307/46*** *(2006.01)*

(86) Internationale Anmeldenummer:
**PCT/EP2012/075059**

(87) Internationale Veröffentlichungsnummer:
**WO 2013/087614 (20.06.2013 Gazette 2013/25)**

(54) **HERSTELLUNG VON 5-HYDROXYMETHYLFURFURAL (HMF) AUS SACCHARIDLÖSUNGEN IN GEGENWART EINES LÖSEMITTELS MIT EINEM SIEDEPUNKT GRÖßER 60°C UND KLEINER 200°C (BEI NORMALDRUCK, KURZ LEICHTSIEDER GENANNT)**

PREPARATION OF 5-HYDROXYMETHYLFURFURAL (HMF) FROM SACCHARIDE SOLUTIONS IN THE PRESENCE OF A SOLVENT HAVING A BOILING POINT GREATER THAN 60°C AND LESS THAN 200°C (AT STANDARD PRESSURE, CALLED LOW BOILER FOR SHORT)

PRODUCTION DE 5-HYDROXYMÉTHYLFURFURAL (HMF) À PARTIR DE SOLUTIONS DE SACCHARIDE EN PRÉSENCE D'UN SOLVANT PRÉSENTANT UN POINT D'ÉBULLITION SUPÉRIEUR À 60°C ET INFÉRIEUR À 200 °C (À UNE PRESSION NORMALE, APPELÉ SOLVANT À POINT D'ÉBULLITION BAS)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 EP 11193157**
**17.08.2012 EP 12180908**

(43) Veröffentlichungstag der Anmeldung:
**22.10.2014 Patentblatt 2014/43**

(73) Patentinhaber: **BASF SE**
**67056 Ludwigshafen am Rhein (DE)**

(72) Erfinder:
- **BACKES, René**
**68623 Lampertheim (DE)**
- **BLANK, Benoit**
**68167 Mannheim (DE)**
- **KINDLER, Alois**
**67269 Grünstadt (DE)**
- **FELDNER, Carmen**
**67069 Ludwigshafen (DE)**

(74) Vertreter: **BASF IP Association**
**BASF SE**
**G-FLP-C006**
**67056 Ludwigshafen (DE)**

(56) Entgegenhaltungen:
**EP-A1- 2 033 958**
**CN-A- 102 399 203**
**FR-A1- 2 663 933**
**US-A- 2 929 823**
**US-A- 3 201 331**

- **KUSTER B F M: "5-Hydroxymethylfurfural (HMF). A Review Focussing on its manufacture", STARKE - STARCH, WILEY-VCH VERLAG, WEINHEIM, DE, Bd. 42, Nr. 8, 1. Januar 1990 (1990-01-01) , Seiten 314-321, XP002463454, ISSN: 0038-9056, DOI: 10.1002/STAR.19900420808**
- **JUBEN N CHHEDA ET AL: "Production of 5-hydroxymethylfurfural and furfural by dehydration of biomass-derived mono- and poly-saccharides", GREEN CHEMISTRY, ROYAL SOCIETY OF CHEMISTRY, CAMBRIDGE, GB, Bd. 9, Nr. 4, 1. Januar 2007 (2007-01-01), Seiten 342-350, XP008142504, ISSN: 1463-9262, DOI: 10.1039/B611568C [gefunden am 2007-01-17]**
- **Daniel Bethge: "Reaktivdestillation eröffnet neue Perspektiven", , 30 April 2010 (2010-04-30), XP55235758, Retrieved from the Internet: URL:http://chemanager-online.com [retrieved on 2015-12-11]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 791 125 B1

## Beschreibung

Die vorliegenden Erfindung betrifft ein Verfahren zur Herstellung von 5-Hydroxymethylfurfural (HMF), welches dadurch gekennzeichnet ist, dass

a) wässerige Lösungen (im Nachfolgenden Ausgangs-Lösung genannt), welche

- eine oder mehrere Hexosen und
- ein organisches Lösemittel mit einem Siedepunkt größer 200°C (bei Normaldruck) (kurz Hochsieder genannt) und
- Wasser enthalten

und ein Lösemittel mit einem Siedepunkt größer 60°C und kleiner 200°C (bei Normaldruck, kurz Leichtsieder genannt) einem Reaktionsgefäß zugeführt werden,

b) in dem Reaktionsgefäß eine Umsetzung der Hexose zu HMF in Gegenwart des Leichtsieders bei gleichzeitiger destillativer Abtrennung des HMF erfolgt und

c) als Destillat eine verdünnte HMF, Wasser und Leichtsieder enthaltende Lösung (im Nachfolgenden Destillat genannt) erhalten wird.

Für chemische Synthesen sind zunehmend Verbindungen von Bedeutung, die aus nachwachsenden Rohstoffen erhalten werden und durch chemische Umsetzungen leicht in technisch anwendbare Verbindungen überführt werden können.

In diesem Zusammenhang ist 5-Hydroxymethylfurfural (HMF) bekannt, welches durch unterschiedliche Verfahren aus Hexosen oder anderen Sacchariden hergestellt werden kann. Aus HMF ist z.B. leicht 2,5-Furandicarbonsäure erhältlich, welche als Dicarbonsäure zur Herstellung von Polymeren, wie Polyester oder Polyurethane, geeignet ist, und andere Dicarbonsäuren aus nicht nachwachsenden Rohstoffen in technischen Anwendungen ersetzen kann.

HMF wird in der Regel durch sauer katalysierte Dehydratisierung von Hexosen wie Glucose oder Fructose hergestellt. Als Reaktionsprodukt werden saure Lösungen erhalten, welche neben dem HMF nicht umgesetzte Ausgangsstoffe und/oder Nebenprodukte enthalten. Bei der HMF-Synthese erfolgt in der Regel nur ein Teilumsatz der Ausgangsstoffe, um die Bildung von Nebenprodukten zu vermeiden. Im Allgemeinen enthalten die erhaltenen Lösungen daher nicht umgesetzte Ausgangsstoffe wie Hexosen oder aus Hexosen aufgebaute Oligomere oder Polymere. Bei höheren Umsätzen nimmt die Menge an Nebenprodukten zu.

Die Abtrennung des HMF aus der Reaktionslösung, welche Ausgangsstoffe oder Nebenprodukte der HMF-Synthese enthalten, ist aufwendig und erschwert die Zugänglichkeit von HMF.

So beschreiben Feroz Kabir Kazi et al. in Chem Eng. J. 169 (2011), Seiten 329-338 die Abtrennung des HMF von der sauren Reaktionslösung durch ein aufwendiges Extraktionsverfahren unter Verwendung eines organischen Lösemittels (Butanol); es wird eine Lösung von HMF in Butanol erhalten.

Aus DE-A 3601281 ist ein chromatographisches Abtrennverfahren bekannt, bei dem zunächst jegliche organische Lösemittel entfernt werden und die wässrige HMF-Lösung mit einer Ionenaustauschersäule aufgetrennt wird. Die gewonnene HMF-Fraktion wird kristallisiert.

Eine weitere Methode, HMF aus der Reaktionslösung abzutrennen, ist die Umwandlung des HMF in eine andere, leichter abtrennbare Verbindung, gegebenenfalls gefolgt von einer Rückumwandlung in HMF nach erfolgter Abtrennung. So wird HMF nach Mark Mascal und Edward B. Nikitin in 2008 Angew. Chemie Vol. 47, Seiten 7924-7926 in das stabilere 5-Chlormethylfurfural umgewandelt und anschließend wieder in HMF oder dessen Derivate überführt. Alternativ werden nach EP-A 1834950 die Ether bzw. nach EP-A 1834951 die Ester des HMF hergestellt, welche sich nach erfolgter Abtrennung direkt für weitere Synthesen eignen.

Haru Kawamoto, Shinya Saito et al. beschreiben in J. Wood Sci. (2007), 53, Seiten 127 - 133 die Pyrolyse von Cellulose unter Bildung von Levoglucosenon, Furfural und/oder HMF unter verschiedenen Bedingungen, auch bei Zufuhr von Wasserdampf.

Daniel Bethge : Reaktivdestillation eröffnet neue Perspektiven, 30.4.2010, XP055235758, http:// chemmager-online.com beschreibt ganz allgemein Reaktivdestillationen jedoch ohne Bezugnahme zur HMF-Synthese.

EP2033958 A1 beschreibt ein Herstellungsverfahren von Methoxymethylfurfural. Als eine mögliche Option der Reaktionsführung wird die Reaktivdestillation beschreiben.

In der FR2663933 und der FR2664273 wird beschrieben wie Fructose und Saccharose in einer Schmelze von sauren Salzen ($Na_3PO_4$ und $KH_2PO_4$) unter Einwirkung von überhitztem Wasserdampf zu HMF umgewandelt werden. Ein geringer Teil des HMF wird dabei durch den Wasserdampf mitgerissen, jedoch wird der Großteil des HMF anschließend mittels Extraktion aus der Salzschmelze isoliert.

US4400468 offenbart sie saure Hydrolyse von Biomasse unter Einwirkung von Wasserdampf zu Zuckern und die direkte Umwandlung der in der Mischung vorhandenen Hexose-Anteile zu HMF. In diesem Fall wird das gebildete HMF jedoch nicht in reiner Form isoliert.

HMF sollte für weitere Synthesen in möglichst reiner Form vorliegen. Für weitere Synthesen sind insbesondere wässrige Lösung von HMF geeignet, welche Nebenprodukte oder restliche Ausgangsstoffe nicht oder allenfalls in sehr geringen Mengen enthält. Bisher bekannte Verfahren, HMF oder dessen wässrige Lösungen mit ausreichender Reinheit herzustellen, sind äußerst aufwendig.

CN102399203 und Wei et al. in Green Chem., 2012, 14, Seiten 1220 - 1226 offenbaren ein destillatives Verfahren zur gleichzeitigen Herstellung und Isolierung von HMF durch Abbau von Fructose und Glucose in ionischen Flüssigkeiten. Das Verfahren umfasst die Zugabe eines Saccharids in eine ionische Flüssigkeit basierend auf Imidazolium-Derivaten bevorzugt mit langen Alkylseitenketten (z.B. 1-Methyl-3-octyl imidazolium Chlorid), in Gegenwart eines Co-Katalysators und eines Strippmittels bei 100 bis 500 Pa, einer Reaktionstemperatur von 120 - 180°C und einer Reaktionszeit von 10 bis 60 Minuten. Das Strippmittel ist Stickstoff, ein anderes inertes Gas, Kohlendioxid, ein C1-C8 Alkan, Aceton oder Methyl-Isobutyl-Keton.

Als Co-Katalysator wird ein Metall-Salz oder Metall Oxid eingesetzt, das zur Stabilisierung des gebildeten HMFs dient aber auch als Katalysator für die Isomerisierung von Glucose zu Fructose dient. Eine solche Isomerisierung mit Metallsalzen ist bereits aus der Literatur bekannt (Glucose-isomerization with Chromium-salts - Science 2007, 316, 1597 -1600; Angew. Chem. Int. Ed. 2008, 47, 9345 - 9348; Chem. Eur. J. 2011, 17, 5281-5288; Glucose-isomerization with rare-earth metals - J. Mol. Cat. A, 2012, 356, 158-164; HMF from Glucose with lanthanides - Green Chem., 2010, 12, 321-325; Conversion of Cellulose to Furans with metal salts - J. Mol. Catal. A, 2012, 357, 11-18; Sn-Beta Zeolithes - ACS Catal. 2011, 1, 408-410).

Die Verwendung von ionischen Flüssigkeiten, insbesondere von substituierten Imidazoliumchlorid Derivaten, für die Synthese von HMF aus Fructose und Glucose ist in der Literatur gut bekannt. Jedoch ist nicht nur die Isolierung des gebildeten HMF aus der ionischen Flüssigkeit sehr aufwändig - meist wird die IL mit einem organischen Lösemittel extrahiert, außerdem die Reaktion in ILs läuft deutlich schlechter ab in Anwesenheit von Wasser und daher wird in allen Fällen das Saccharid in möglichst wasserfreier IL umgesetzt. Letztere muss vor einer weiteren Verwendung aufwändig entwässert werden (z.B. Biores. Tech. 2011, 102, 4179-4183).

Der negative Einfluss von Wasser im Reaktionsmedium bei der Umsetzung von Zuckern, insbesondere Fructose und Glucose, zu HMF ist in der Literatur gut beschrieben (z.B. Carbohydr. Res. 1977, 54, 177-183; Science 2007, 136, 1597-1600). Wie dem Fachmann wohl bekannt ist, wird durch Wasser einerseits die Dehydratisierungsreaktion der Zucker verlangsamt und außerdem die Rehydratisierung und Spaltung des gebildeten HMF in Ameisensäure und Lävulinsäure gefördert (zum postulierten Mechanismus der Reaktion siehe Science 2006, 312, 1933-1937).Wie in Beispiel 1 gezeigt wird, läuft die Reaktion mit steigendem Wassergehalt deutlich langsamer ab und liefert geringere Ausbeuten an HMF.

Aufgabe der vorliegenden Erfindung war daher ein großtechnisches Verfahren, mit dem HMF in möglichst einfacher und effektiver Weise hergestellt werden kann, HMF gleichzeitig in möglichst reiner Form erhalten wird und HMF daher von umgesetzten Ausgangstoffen oder Nebenprodukten der Synthese unmittelbar möglichst vollständig abgetrennt wird.

In großtechnischen Verfahren soll die Umsetzung in einem technisch wie ökonomisch vorteilhaften Temperatur und Druckbereich bei hohem Umsatz und hoher Raum-Zeit-Ausbeute durchgeführt werden. Insbesondere die Verwendung von kommerziell verfügbaren wässrigen Saccharidlösungen in unterschiedlichen Konzentrationen im Gegensatz zu kristallinen Sacchariden ist von hoher Bedeutung. Desweiteren sollten die Einsatzstoffe möglichst geringe Ansprüche an die Apparaturen und die Verfahrensweise stellen und eine einfach Aufreinigung und Isolierung des Wertprodukts ermöglichen. Die Verwendung eines Gases als Strippmittel bei einer Reaktions-führung im Vakuum ist großtechnisch schwer durchzuführen, da eine hohe Pumpleistung erforderlich ist, damit das gewünschte Vakuum gehalten werden kann. Bei der großtechnischen Verwendung von organischen Lösemitteln als Strippmittel sind deren hohe Kosten und aufwändige Rückgewinnung nachteilhaft. Bei der Herstellung von HMF sind desweiteren geringe Verweilzeiten bei hohen Temperaturen durch die Instabilität des Produkts von großem Vorteil. Insbesondere eine schnelle Abtrennung des gebildeten HMF aus der Reaktionslösung ermöglicht höhere Ausbeuten. Ein wesentlicher Faktor bei großtechnischen Verfahren ist die Komplexität des Verfahrens, so sind beispielsweise sogenannte Eintopfverfahren aus unterschiedlichen Gründen zu bevorzugen. Eine besonders bevorzugte Verfahrensvariante ist die Reaktivdestillation, bei der in einem Eintopfverfahren das Zielprodukt gebildet und gleichzeitig aus der Reaktionslösung abgetrennt wird.

Demgemäß wurde das eingangs definierte Verfahren gefunden.

Bei dem erfindungsgemäßen Verfahren wird HMF in Gegenwart eines Lösemittels mit einem Siedepunkt größer 60°C und kleiner 200°C (bei Normaldruck, kurz Leichtsieder genannt) hergestellt und unmittelbar von Nebenprodukten und nicht umgesetzten Ausgangsstoffen der HMF-Synthese abgetrennt. Dieses Verfahren kann im Gegensatz zum Stand der Technik im großtechnischen Maßstab durchgeführt werden.

Zu Verfahrensschritt a)

Im Verfahrensschritt a) werden Lösungen (im Nachfolgenden Ausgangs-Lösung genannt), welche

- eine oder mehrere Hexosen und
- ein organisches Lösemittel mit einem Siedepunkt größer 200°C (bei Normaldruck) (kurz Hochsieder genannt), und
- Wasser enthalten

und ein Lösemittel mit einem Siedepunkt größer 60°C und kleiner 200°C (bei Normaldruck, kurz Leichtsieder genannt) einem Reaktionsgefäß zugeführt.

Bei dem Saccharid handelt es sich um Hexosen oder aus Hexosen aufgebaute Oligomere oder Polymere. Bei den Hexosen handelt es sich vorzugsweise um Fructose, Glucose oder Gemische von Fructose und Glucose. Besonders bevorzugt handelt es sich um Fructose oder Gemische von Fructose mit Glucose. Ganz besonders bevorzugt handelt es sich bei der Hexose um Fructose.

Die Ausgangs-Lösung kann auch Nebenprodukte oder Ausgangsprodukte aus der Herstellung der Saccharide enthalten. Zum Beispiel können Saccharide durch Abbau von Polymeren wie Cellulose oder Stärke gewonnen werden. Daher kann die Ausgangs-Lösung noch Restmengen an derartigen Polymeren oder deren oligomere Abbauprodukte enthalten.

Vorzugsweise enthält die Ausgangs-Lösung 1 bis 60 Gew. % Saccharid, besonders bevorzugt 10 bis 50 Gew. % Saccharid, bezogen auf das Gesamtgewicht der Ausgangs-Lösung.

Vorzugsweise enthält die Ausgangslösung weniger als 10 Gew. %, insbesondere weniger als 5 Gew. % und besonders bevorzugt weniger als 1 Gew. % an Nebenprodukten oder Ausgangsstoffen aus der Herstellung von Sacchariden (bezogen auf das Gesamtgewicht der Ausgangslösung). Insbesondere ist die Ausgangs-Lösung im Wesentlichen frei von Nebenprodukten und Ausgangsstoffen aus der Herstellung von Sacchariden.

In einer alternativen Ausführungsform kann die Ausgangslösung auch Metallchloride oder Metallnitrate der allgemeinen Formel MXn als Isomerisierungssalze enthalten, wobei M ein Metall, X Chlor oder Nitrat und n eine ganze Zahl von 1 bis 4 ist. Diese Isomerisierungssalze sind bevorzugt dann in der Ausgangslösung enthalten, wenn das Saccharid der Ausgangslösung Glucose ist oder ein Saccharid, das Glucose-Einheiten enthält wie beispielsweise Saccharose. Die Verwendung von Isomerisierungssalzen ist bereits in der Literatur beschrieben (Science 2007, 316, 1597-1600, Carbohydr. Pol. 2012, 90, 792-798, Chem. Eur. J. 2011, 17, 5281-5288, Green Chem. 2009, 11, 1746-1749) Bevorzugt werden Metallchloride oder Metallnitrate ausgewählt aus der Gruppe $CrCl_2$, $CrCl_3$, $AlCl_3$, $FeCl_2$, $FeCl_3$, CuCl, $CuCl_2$, CuBr, $VCl_3$, $MoCl_3$, $PdCl_2$, $PtCl_2$, $RuCl_3$, $RhCl_3$, $Ni(NO_3)_2$, $Co(NO_3)_2$, $Cr(NO_3)_3$, $SnCl_4$ verwendet. Ganz besonders bevorzugt sind $CrCl_2$ und $CrCl_3$.

Die Ausgangs-Lösung enthält weiterhin ein organisches Lösemittel mit einem Siedepunkt größer 200°C (bei Normaldruck), insbesondere größer 250°C (im Nachfolgenden kurz Hochsieder genannt).

Als Hochsieder in Betracht kommen hydrophile Lösemittel; es kann sich um protische, hydrophile organische Lösemittel, z. B. Alkohole, oder aprotische hydrophile Lösemittel, z. B. Ether oder Ketone, wie Dimethylsulfoxid, handeln. In Betracht kommen auch ionische Flüssigkeiten, schwersiedende Öle wie beispielsweise Paraffine und hochsiedende Ester wie beispielsweise Hexamoll DINCH (1,2-Cyclohexandicarbonsäurediisononylester). Weitere mögliche Hochsieder sind: 1,3-Dimethylpropylen Harnstoff (DMPU), Tri-n-octylphosphinoxid (TOPO), Hexamethylorthophosphorsäure Triamid (HMPT), 3-Methyl-2-oxazolidon, 2-Oxazolidon, o-Dihydroxybenzol, Catechol, N,N-Dibutylharnstoff und Dibutylsulfon.

Im Rahmen dieser Erfindung bevorzugte Hochsieder sind Polyether und ionische Flüssigkeiten.

Polyether

Die Polyether haben vorzugsweise einen Schmelzpunkt kleiner 60°C, insbesondere kleiner 30°C (bei Normaldruck, 1 bar); besonders bevorzugte Polyether sind bei 20°C (Normaldruck) flüssig.

Die Polyether enthalten mindestens zwei Ethergruppen. Vorzugsweise enthalten die Polyether mindestens 3, insbesondere mindestens 4, besonders bevorzugt mindestens 6 Ethergruppen. Im Allgemeinen enthalten sie nicht mehr als 40, insbesondere nicht mehr als 30 Ethergruppen, besonders bevorzugt nicht mehr als 20 Ethergruppen.

In einer besonderen Ausführungsform enthalten die Polyether keine Heteroatome außer Sauerstoff in Form von Ethergruppen und gegebenenfalls Hydroxylgruppen.

Insbesondere handelt es sich um aliphatische Polyether, besonders bevorzugte Polyether sind Polyalkylenglycole, wobei die endständigen Hydroxylgruppen mit Alkylgruppen, insbesondere C1- bis C4-Alkylgruppen verethert sein können.

Bei den Alkylengruppen der Polyalkylenglycole kann es sich z.B. um C2- bis C10-, insbesondere um C2- bis C4-Alkylengruppen, wie Ethylen-, Propylen- oder Butylengruppen handeln. Die Polalkylenglycole können auch verschiedene Alkylengruppen enthalten, z.B. in Form von Blöcken.

Ganz besonders bevorzugt sind daher Poly-C2-bis C4-alkylenglycole, insbesondere Polyethylenglycol, deren endständige Hydroxylgruppen gegebenenfalls mit Alkylgruppen verethert sein können; die Anzahl der wiederkehrenden Alkylenethergruppen entspricht der vorstehenden Anzahl der Ethergruppen, insbesondere ist die Anzahl der wiederkehrenden Alkylenethergruppen 4 bis 30, besonders bevorzugt 6 bis 20. Die endständigen Hydroxylgruppen der Polyalkylenglycole können mit Alkylgruppen, insbesondere C1-bis C4-Alkylgruppen, verethert sein.

Ionische Flüssigkeit (IL)

Ionische Flüssigkeiten bezeichnen im Rahmen der vorliegenden Anmeldung organische Salze, die bereits bei Temperaturen unterhalb 180 °C flüssig sind. Vorzugsweise besitzen die ionischen Flüssigkeiten einen Schmelzpunkt von weniger als 150 °C, besonders bevorzugt weniger als 120 °C, insbesondere weniger als 100 °C.

Ionische Flüssigkeiten, die bereits bei Raumtemperatur in flüssigem Aggregatszustand vorliegen, werden beispielsweise von K. N. Marsh et al., Fluid Phase Equilibria 219 (2004), 93 - 98 und J. G. Huddleston et al., Green Chemistry 2001, 3, 156 - 164 beschrieben.

Für den Einsatz in dem erfindungsgemäßen Verfahren geeignete ionische Flüssigkeiten sind in der WO 2008/090155 (S.4, Zeile 38 bis S. 37, Zeile 31)und WO 2008/090156 (S. 7, Zeile 1 bis S. 39, Zeile 37) beschrieben, worauf hier Bezug genommen wird.

In der ionischen Flüssigkeit liegen Kationen sowie Anionen vor. Dabei kann innerhalb der ionischen Flüssigkeit vom Kation ein Proton oder ein Alkylrest an das Anion übertragen werden, wodurch zwei neutrale Moleküle resultieren. In der erfindungsgemäß eingesetzten ionischen Flüssigkeit kann also ein Gleichgewicht von Anionen, Kationen sowie daraus gebildeten neutralen Molekülen vorliegen.

Bevorzugte ionische Flüssigkeiten sind Kombinationen aus stickstoffhaltigen Kationenkomponenten (wie Imidazolioliumderivaten) und Halogenionen als Anionen.

Geeignete Verbindungen, die sich zur Bildung des Kations von ionischen Flüssigkeiten eignen, sind z. B. in der DE 102 02 838 A1 ([0030] bis [0073]) beschrieben. Diese Verbindungen enthalten vorzugsweise wenigstens ein Heteroatom, wie z. B. 1 bis 10 Heteroatome, die vorzugsweise ausgewählt sind unter Stickstoff-, Sauerstoff-, Phosphor- und Schwefelatomen. Bevorzugt sind Verbindungen, die wenigstens ein Stickstoffatom und gegebenenfalls zusätzlich wenigstens ein weiteres, von Stickstoff verschiedenes Heteroatom enthalten. Bevorzugt sind Verbindungen, die mindestens ein Stickstoffatom, besonders bevorzugt 1 bis 10 Stickstoffatome, insbesondere 1 bis 5 Stickstoffatome, ganz besonders bevorzugt 1 bis 3 Stickstoffatome und speziell 1 oder 2 Stickstoffatome enthalten. Die letztgenannten Stickstoffverbindungen können weitere Heteroatome wie Sauerstoff-, Schwefel- oder Phosphoratome enthalten.

Bevorzugt sind solche Verbindungen, die mindestens einen fünf- bis sechsgliedrigen Heterocyclus, insbesondere einen fünfgliedrigen Heterocyclus, enthalten, der mindestens ein Stickstoffatom sowie gegebenenfalls ein Sauerstoff- oder Schwefelatom aufweist. Besonders bevorzugt sind solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit zwei Stickstoffatomen. Weiterhin bevorzugt sind aromatische Heterocyclen.

Bevorzugt als Kationen sind unsubstituierte oder substituierte Imidazoliumionen. Besonders geeignete Imidazoliumionen sind 1-Methylimidazolium, 1-Ethylimidazolium, 1-(1-Propyl)-imidazolium, 1-(1-Allyl)-imidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1,3-Diethylimidazolium, 1-Ethyl-3-methylimidazolium, 1-(1-Butyl)-3-methylimidazolium1-(1-Butyl)-3-ethylimidazolium, 1-(1-Hexyl)-3-methyl-imidazolium, 1-(1-Hexyl)-3-ethyl-imidazolium, 1-(1-Hexyl)-3-butyl-imidazolium, 1-(1-Octyl)-3-methylimidazolium, 1-(1-Octyl)-3-ethylimidazolium, 1-(1-Octyl)-3-butylimidazolium, 1-(1-Dodecyl)-3-methylimidazolium, 1-(1-Dodecyl)-3-ethylimidazolium, 1-(1-Dodecyl)-3-butylimidazolium, 1-(1-Dodecyl)-3-octylimidazolium, 1-(1-Tetradecyl)-3-methylimidazolium, 1-(1-Tetradecyl)-3-ethylimidazolium, 1-(1-Tetradecyl)-3-butylimidazolium, 1-(1-Tetradecyl)-3-octylimidazolium, 1-(1-Hexadecyl)-3-methylimidazolium, 1-(1-Hexadecyl)-3-ethyl-imidazolium, 1-(1-Hexadecyl)-3-butylimidazolium, 1-(1-Hexadecyl)-3-octylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium, 1-(1-Octyl)-2,3-dimethylimidazolium, 1,4-Dimethylimidazolium, 1,3,4-Trimethylimidazolium, 1,4-Dimethyl-3-ethylimidazolium, 3-Methylimidazolium, 3-Ethylimidazolium, 3-n-Propylimidazolium, 3-n-Butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium, 1-Prop-1-en-3-yl-3-methylimidazolium und 1-Prop-1-en-3-yl-3-butylimidazolium. Speziell geeignete Imidazoliumionen (IVe) sind 1,3-Diethylimidazolium, 1-Ethyl-3-methylimidazolium, 1-(n-Butyl)-3-methylimidazolium.

Das Anion der ionischen Flüssigkeit ist beispielsweise ausgewählt aus

1) Anionen der Formeln: F-, $Cl^-$, $Br^-$, $I^-$, $BF_4^-$, $PF_6^-$, $CF_3SO_3^-$, $(CF_3SO_3)_2N^-$, $CF_3CO_2^-$, $CCl_3CO_2^-$, CN-, SCN-, OCN-.

2) Anionen der Formeln: $SO_4^{2-}$, $HSO_4^-$, $SO_3^{2-}$, $HSO_3^-$, $R^cOSO_3^-$, $R^cSO_3^-$.
3) Anionen der Formeln: $PO_4^{3-}$, $HPO_4^{2-}$, $H_2PO_4^-$, $R^cPO_4^{2-}$, $HR^cPO_4^-$, $R^cR^dPO_4^-$.
4) Anionen der Formeln: $R^cHPO_3^-$, $R^cR^dPO_2^-$, $R^cR^dPO_3^-$.
5) Anionen der Formeln: $PO_3^{3-}$, $HPO_3^{2-}$, $H_2PO_3^-$, $R^cPO_3^{2-}$, $R^cHPO_3^-$, $R^cR^dPO_3^-$.
6) Anionen der Formeln: $R^cR^dPO_2^-$, $R^cHPO_2^-$, $R^cR^dPO^-$, $R^cHPO^-$.
7) Anionen der Formel $R^cCOO^-$.
8) Anionen der Formeln: $BO_3^{3-}$, $HBO_3^{2-}$, $H_2BO_3^-$, $R^cR^dBO_3^-$, $R^cHBO_3^-$, $R^cBO_3^{2-}$, $B(OR^c)(OR^d)(OR^e)(ORf)^-$, $B(HSO_4)_4^-$, $B(R^cSO_4)_4^-$.
9) Anionen der Formeln: $R^cBO_2^{2-}$, $R^cR^dBO^-$.
10) Anionen der Formeln: $HCO_3^-$, $CO_3^{2-}$, $R^cCO_3^-$
11) Anionen der Formeln: $SiO_4^{4-}$, $HSiO_4^{3-}$, $H_2SiO_4^{2-}$, $H_3SiO_4^-$, $R^cSiO_4^{3-}$, $R^cR^dSiO_4^{2-}$, $R^cR^dR^eSiO_4^-$, $HR^cSiO_4^{2-}$, $H_2R^cSiO_4^-$, $HR^cR^dSiO_4^-$
12) Anionen der Formeln: $R^cSiO_3^{3-}$, $R^cR^dSiO_2^{2-}$, $R^cR^dR^eSiO^-$, $R^cR^dR^eSiO_3^-$, $R^cR^dR^eSiO_2^-$, $R^cR^dSiO_3^{2-}$.
13) Anionen der Formeln:

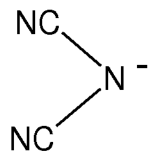

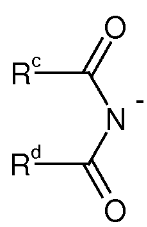

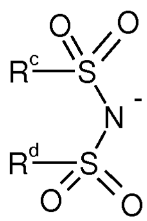

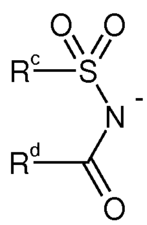

.

14) Anionen der Formeln:

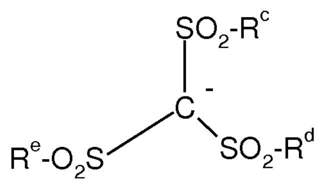

15) Anionen der Formel $R^cO^-$.
16) Anionen der Formeln HS-, $[Sv]^{2-}$, [HSv]-, $[R^cS]^-$, wobei v eine ganze positive Zahl von 2 bis 10 ist.

Die Reste $R^c$, $R^d$, Re und $R^f$ stehen vorzugsweise unabhängig voneinander für

- Wasserstoff;
- unsubstituiertes oder substituiertes Alkyl, vorzugsweise unsubstituiertes oder substituiertes C1-C30-Alkyl, besonders bevorzugt unsubstituiertes oder substituiertes C1-C18-Alkyl, das durch wenigstens ein Heteroatom oder heteroatomhaltige Gruppe unterbrochen sein kann;
- unsubstituiertes oder substituiertes Aryl, vorzugsweise unsubstituiertes oder substituiertes C6-C14-Aryl, besonders bevorzugt unsubstituiertes oder substituiertes C6-C10-Aryl;
- unsubstituiertes oder substituiertes Cycloalkyl, vorzugsweise unsubstituiertes oder substituiertes C5-C12-Cycloalkyl;
- unsubstituiertes oder substituiertes Heterocycloalkyl, vorzugsweise unsubstituiertes oder substi-tuiertes Heterocycloalkyl mit 5 oder 6 Ringatomen, wobei der Ring neben Kohlenstoffringatomen 1, 2 oder 3 Heteroatome oder heteroatomhaltige Gruppen aufweist;
- unsubstituiertes oder substituiertes Heteroaryl, vorzugsweise unsubstituiertes oder substituiertes Heteroaryl mit 5 bis 10 Ringatomen, wobei der Ring neben Kohlenstoffringatomen 1, 2 oder 3 Heteroatome oder heteroatomhaltige Gruppen aufweist, die ausgewählt sind unter Sauerstoff, Stickstoff, Schwefel und NRa; wobei in Anionen, die mehrere

Reste $R^c$ bis $R^f$ aufweisen, auch jeweils zwei dieser Reste zusammen mit dem Teil des Anions, an das sie gebunden sind, für wenigstens einen gesättigten, ungesättigten oder aromatischen Ring oder ein Ringsystem mit 1 bis 12 Kohlenstoffatomen stehen können, wobei der Ring oder das Ringsystem 1 bis 5 nicht benach-barte Heteroatome oder heteroatomhaltige Gruppen aufweisen kann, die vorzugsweise ausgewählt sind unter Sauerstoff, Stickstoff, Schwefel und NRa, und wobei der Ring oder das Ringsystem unsubstituiert ist oder substituiert sein kann.

Bevorzugte Anionen sind $Cl^-$, $Br^-$, Formiat, Acetat, Propionat, Butyrat, Lactat, Saccharinat, Carbonat, Hydrogencarbonat, Sulfat, Sulfit, C1-C4-Alkylsulfate, Methansulfonat, Tosylat, Trifluoracetat, C1-C4-Dialkylphosphate und Hydrogensulfat.

Besonders bevorzugte Anionen sind $Cl^-$, $Br^-$, $HCOO^-$, $CH_3COO^-$, $CH_3CH_2COO^-$, Carbonat, Hydrogencarbonat, Sulfat, Sulfit, Tosylat, $CH_3SO_3^-$ oder $CH_3OSO_3^-$Insbesondere sind die Anionen ausgewählt unter $Cl^-$ und $CH_3SO_3^-$.

Geeignete ionische Flüssigkeiten für den Einsatz in dem erfindungsgemäßen Verfahren sind kommerziell erhältlich, z. B. unter dem Markennamen Basionic® der BASF SE.

Vorteilhaft für einen Einsatz in dem erfindungsgemäßen Verfahren sind Imidazoliumchloride oder Imidazoliummethansulfonate oder Mischungen davon.

Weiterhin vorteilhaft für einen Einsatz in dem erfindungsgemäßen Verfahren sind z. B.: 1-Ethyl-3-methylimidazoliumchlorid (EMIM Cl, Basionic ST 80), 1-Ethyl-3-methylimidazolium-methansulfonat (EMIM $CH_3SO_3$, Basionic ST 35), 1-Butyl-3-methylimidazoliumchlorid (BMIM Cl, Basionic ST 70), 1-Butyl-3-methylimidazoliummethansulfonat (BMIM $CH_3SO_3$, Basionic ST 78), Methylimidazoliumchlorid (HMIM Cl, Basionic AC 75), Methyl-imidazoliumhydrogensulfat (HMIM $HSO_4$ Basionic AC 39), 1-Ethyl-3-methylimidazolium-hydrogensulfat (EMIM $HSO_4$ Basionic AC 25), 1-Butyl-3-methylimidazoliumhydrogensulfat (BMIM $HSO_4$ Basionic AC 28) 1-Ethyl-3-methylimidazoliumacetat (EMIM Acetat, Basionic BC 01), 1-Butyl-3-methylimidazoliumacetat (BMIM Acetat, Basionic BC 02).

Besonders bevorzugt sind 1-Ethyl-3-methylimidazoliumchlorid, 1-Butyl-3-methylimidazoliumchlorid, Methylimidazoliumchlorid, 1-Ethyl-3-methylimidazoliummethansulfonat, 1-Butyl-3-methylimidazolium-methansulfonat und Mischungen davon.

Ganz besonders bevorzugt sind 1-Ethyl-3-methylimidazoliumchlorid (EMIM Cl, Basionic ST 80), 1-Butyl-3-methylimidazoliumchlorid (BMIM Cl, Basionic ST 70) und 1-Ethyl-3-methylimidazolium-methansulfonat (EMIM $CH_3SO_3$, Basionic ST 35).

Die Ausgangs-Lösung enthält neben dem Hochsieder mindestens ein weiteres Lösemittel. Bei dem weiteren Lösemittel handelt es sich insbesondere um Wasser. Es kann sich auch um Gemische von Wasser mit hydrophilen organischen Lösemitteln mit einem Siedepunkt kleiner 200°C (Niedrigsieder genannt) handeln, in denen das verwendete Saccharid vorzugsweise löslich sein sollte.

Besonders bevorzugt handelt es sich bei dem weiteren Lösemittel um Wasser. Die Ausgangs-Lösung ist daher besonders bevorzugt eine wässrige Lösung.

In einer besonderen Ausführungsform enthält die Ausgangslösung als Lösemittel ausschließlich Wasser und den Hochsieder.

Die Ausgangs-Lösung enthält den Hochsieder, insbesondere den Polyether oder die ionische Flüssigkeit, vorzugsweise in Mengen von 5 bis 90 Gew. %, insbesondere von 30 bis 80 Gew.%, besonders bevorzugt von 40 bis 80 Gew. %, bezogen auf das Gesamtgewicht der Ausgangs-Lösung.

Vorzugsweise ist der Gehalt der Ausgangs-Lösung an Wasser kleiner 60 Gew. %, insbesondere kleiner 50 Gew. % und besonders bevorzugt kleiner 40 Gew.% bezogen auf das Gesamtgewicht der Ausgangslösung.

Die Ausgangs-Lösung enthält weiterhin vorzugsweise einen Katalysator. Der Katalysator katalysiert die Umsetzung des Saccharids zu HMF. Als Katalysator sind Säuren geeignet. Als Säuren in Betracht kommen heterogene Säuren, welche in der Ausgangs-Lösung dispergiert sind oder homogene Säuren, welche in der Ausgangs-Lösung gelöst sind. Als homogene Säuren kommen beliebige anorganische oder organische Säuren in Betracht. Besonders bevorzugt werden homogene protische Säuren eingesetzt. Exemplarisch genannt seien para-Toluolsulfonsäure, Methansulfonsäure ($MeOSO_3H$), Oxalsäure, Schwefelsäure, Salzsäure oder Phosphorsäure.

Wird als Hochsieder eine ionische Flüssigkeit oder DMSO verwendet, ist der Einsatz eines Katalysators nicht unbedingt zwingend notwendig, insbesondere bei Halogenidhaltigen ILs wie beispielsweise EMIMCl und BMIMCl. Durch den Einsatz eines Katalysators wird die Reaktion jedoch beschleunigt und damit die Verweilzeit in Verfahrensschritt b) reduziert. Somit wird bei Verwendung von ionischen Flüssigkeiten oder DMSO als Hochsieder vorzugsweise ein Katalysator eingesetzt.

Wird ein Polyether, inbesondere Polyethylenglycol als Hochsieder verwendet ist der Einsatz eines Katalysators zwingend notwendig.

Die Ausgangs-Lösung enthält die Säure vorzugsweise in Mengen von 0,1 bis 10 mol%, besonders bevorzugt von 0,1 bis 5 mol% (bezogen auf das Saccharid).

Bevorzugte Ausgangs-Lösungen enthalten z. B.

1 bis 40 Gew. % Saccharid
5 bis 90 Gew. % Hochsieder, vorzugsweise Polyether oder ionische Flüssigkeit
1 bis 50 Gew. % Wasser
0,1 bis 10 mol % Säure (bezogen auf das Saccharid)
0 bis 10 Gew. % sonstige Bestandteile, z. B. Nebenprodukte aus der Synthese des Saccharids,

bezogen auf das Gesamtgewicht der Lösung.

Besonders bevorzugte Ausgangs-Lösungen enthalten z. B.

5 bis 30 Gew. % Saccharid
30 bis 80 Gew. % Hochsieder, vorzugsweise Polyether oder ionische Flüssigkeit
10 bis 50 Gew. % Wasser
0,1 bis 5 mol % Säure (bezogen auf das Saccharid)
0 bis 5 Gew. % sonstige Bestandteile, z. B. Nebenprodukte aus der Synthese des Saccharids,

bezogen auf das Gesamtgewicht der Lösung.

Die Ausgangs-Lösung kann hierbei auf verschiedene Art und Weise hergestellt werden. In einer Ausführungsform werden die Komponenten der Ausgangs-Lösung einem Reaktionsgefäß zugeführt und dort vorgemischt. Diese Ausgangs-Lösung wird dann dem Verfahrensschritt b) zugeführt. Dabei kann diese Ausgangslösung in einer besonderen Ausführungsform vorher auch auf eine Temperatur von 150-200°C vorgeheizt werden.

In einer alternativen Ausführungsform wird die Ausgangs-Lösung durch folgende Schritte hergestellt:

a1) Das Saccharid und Wasser, liegen in einem Reaktionsgefäß vor.
a2) In einem weiteren Reaktionsgefäß wird der Hochsieder und vorzugsweise der Katalysator vorgelegt
a3) Unmittelbar vor dem Verfahrensschritt b) werden die Komponenten gemischt. Dies erfolgt vorzugsweise in einer Mischkammer.

In einer weiteren alternativen Ausführungsform kann neben dem Hochsieder und vorzugsweise dem Katalysator auch ein Metallchlorid oder Metallnitrat im Verfahrensschritt a2) vorgelegt werden. Bevorzugt wird ein Metallchlorid oder Metallnitrat vorgelegt, wenn das in Verfahrensschritt a1) verwendete Saccharid Glucose ist oder das verwendete Saccharid Glucose-Einheiten enthält wie beispielsweise Saccharose.

Unmittelbar vor dem Verfahrensschritt b) bedeutet, dass der Zeitraum von Beginn der Mischzeit in Verfahrensschritt a) bis zum Eintritt der Mischung in das Reaktionsgefäß, vorzugsweise ein Verdampfer, höchstens 5 Minuten, besonders bevorzugt höchstens eine Minute beträgt.

In einer alternativen Ausführungsform kann die gemischte Ausgangslösung zusätzlich durch ein schlagartiges Druckgefälle (Übergang von Normaldruck in ein Vakuum mittels eines Überströmers) teilweise verdampft und in die Gasphase überführt (sog. "Flashen").

In einer weiteren alternativen Ausführungsform können die Lösungen der Schritte a1 und a2 in den beiden Reaktionsgefäßen getrennt voneinander vor dem Mischen beispielsweise über einen Wärmetauscher auf eine Temperatur zwischen 150 und 200°C vorgeheizt werden.
In diesem Fall liegt die wässrige Ausgangslösung bereits in einem überkritischen Zustand vor und kann in einer weiteren alternativen Ausführungsform durch ein schlagartiges Druckgefälle (Übergang von Überdruck in ein Vakuum mittels eines Überströmers) besonders gut verdampft und ein größerer Wasseranteil in die Gasphase überführt (sog. "Flashen") werden.

Im Falle von Hochsiedern, die bei Raumtemperatur fest oder auch sehr viskos sind, kann eine Beheizung der Vorlagegefäße nötig sein, damit diese vorab aufgeschmolzen werden und pumpbar sind.

Die vorstehend beschriebene Ausgangs-Lösung und das Lösemittel mit einem Siedepunkt größer 60°C und kleiner 200°C (bei Normaldruck, kurz Leichtsieder genannt) werden einem Reaktionsgefäß zugeführt.

Zu Verfahrenschritt b)

In Verfahrenschritt b) erfolgt die Umsetzung der Ausgangs-Lösung zu HMF in Verbindung mit einer für sich genommen bekannten Destillation in Gegenwart eines Lösemittels mit einem Siedepunkt größer 60°C und kleiner 200°C (bei Normaldruck, kurz Leichtsieder genannt). Dazu wird die Ausgangs-Lösung mit dem Leichtsieder im Reaktionsgefäß in Kontakt gebracht Bevorzugte Leichtsieder sind Wasserdampf, Alkohole wie beispielsweise Methanol, Ethanol, 2-Butanol, Mono-, Di- und Polyether wie Ethylenglycol Dimethylether, Diethylenglykolmonomethylether, Triethylenglycol, Ketone wie 2-Butanon oder Methyl-isobutylketon, Ester wie Butylacetat und Aromaten wie Toluol oder Xylol. Bevorzugt sind polar-protische Leichtsieder, die eine gute Wechselwirkung mit HMF ermöglichen. Besonders bevorzugt werden

als Leichtsieder Wasserdampf, Methanol und 2-Butanol eingesetzt. Ganz besonders bevorzugt ist Wasserdampf.

Die Behandlung der Ausgangs-Lösung mit dem Leichtsieder erfolgt vorzugsweise bei vermindertem Druck, in Betracht kommt insbesondere ein Druck von 10 bis 200 mbar. Bevorzugt beträgt der Druck im Reaktionsgefäß 10 bis 100 mbar, besonders bevorzugt 20 bis 80 mbar.

Die Behandlung der Ausgangs-Lösung mit dem Leichtsieder erfolgt vorzugsweise bei einer Temperatur der Ausgangs-Lösung von 100 bis 250°C, besonders bevorzugt von 140 bis 250°C und besonders bevorzugt von 160 bis 220°C und ganz besonders bevorzugt 170 bis 220°C.

Wird als Hochsieder ein Polyethylenglycol verwendet, erfolgt die Behandlung der Ausgangslösung mit dem Leichtsieder vorzugsweise bei einer Temperatur von 160 bis 220°C.

Wird als Hochsieder eine ionische Flüssigkeit verwendet, erfolgt die Behandlung der Ausgangslösung mit dem Leichtsieder vorzugsweise bei einer Temperatur von 180 bis 220°C.

Wird als Leichtsieder Wasserdampf verwendet, erfolgt die Behandlung der Ausgangslösung mit Wasserdampf vorzugsweise bei einer Temperatur von 140 bis 220°C, besonders bevorzugt bei 160 bis 220°C und ganz besonders bevorzugt bei 180 bis 220°C. Bei diesen Temperaturen kann die Behandlung bei technisch sinnvoller, aber minmaler Wasserdampfmenge erfolgen. Durch Erhöhung der Wasserdampfmenge kann die Trennleistung der Reaktivdestillation bei gleichbleibender oder niedriger Temperatur zwar erhöht oder konstant gehalten werden, jedoch ist der Einsatz einer erhöhten Wasserdampfmenge technisch weniger bevorzugt, da ein erhöhter Aufwand betrieben werden muss, um den Druck konstant zu halten, eine höhere Kühlleistung auf der Destillatseite nötig ist und die Kosten für Dampf steigen.

In einer weiteren alternativen Ausführungsform wird kein Leichtsieder zum Reaktionsgefäß zugeführt. In dieser Ausführungsform entsteht der Leichtsieder in Form von Wasserdampf im Reaktionsgefäß aus dem Wasser der Ausgangslösung.

Vorzugsweise wird das erfindungsgemäße Verfahren kontinuierlich betrieben.

Dazu wird die Ausgangs-Lösung und der Leichtsieder dem Reaktionsgefäß kontinuierlich zugeführt und das erhaltene Produkt, bzw. Destillat, kontinuierlich abgeführt.

Die Volumenströme hängen von der Größe, der Reaktorleistung und der Trennleistung des gewählten Reaktionsgefäßes ab.

In einer bevorzugten Ausführungsform liegt das Verhältnis der zugeführten Menge an Leichtsieder zur Menge der zugeführten Ausgangs-Lösung in einem Bereich von 0,2 bis 4 Gewichtseinheiten Leichtsieder auf 1 Gewichtseinheit Ausgangs-Lösung, besonders bevorzugt im Bereich von 0,3 bis 2 Gewichtseinheiten Leichtsieder auf 1 Gewichtseinheit Ausgangs-Lösung und insbesondere 0,3 bis 1,5 Gewichtseinheiten Leichtsieder auf 1 Gewichtseinheit Ausgangs-Lösung.

Als Reaktionsgefäß eignen sich übliche Verdampfer, welche für die Zufuhr von Ausgangs-Lösung und Leichtsieder und insbesondere für vorstehend beschriebene kontinuierliche Verfahrensweise eingerichtet sind.

Als Verdampfer wird vorzugsweise ein Verdampfer mit kurzer Verweilzeit im Sekunden bis Minutenbereich (2 Sekunden bis 10 Minuten) eingesetzt. Vorteilhafterweise wird somit eine geringe thermische Belastung der gebildeten Dehydratisierungsprodukte erreicht. Die Verweilzeit hat einen starken Einfluss auf die Ausbeute an HMF, da bei bereits vollständigem Umsatz durch eine erhöhte Verweilzeit die Destilationsaubeute an HMF stark zurückgeht.

Vorzugsweise beträgt daher die Verweilzeit 1 bis 120 Sekunden, besonders bevorzugt 1 bis 60 Sekunden, ganz besonders bevorzugt 5 bis 30 Sekunden.

Als Verdampfer eignet sich prinzipiell eine dafür übliche Vorrichtung die im einfachsten Fall ein Behältnis oder Rohre mit beheizbaren Wänden als Wärmeübertragungsflächen umfasst. Der Verdampfer kann in geeigneter Weise von außen über die Wände mit Wärme versorgt werden, beispielsweise mit Dampf. Die Temperatur im Verdampfer liegt vorzugsweise in einem Bereich von 100 bis 300 °C, besonders bevorzugt in einem Bereich von 150 °C bis 250 °C. Der Druck im Verdampfer beträgt vorzugsweise von höchstens 100 mbar. Der Druck im Verdampfer liegt besonders bevorzugt in einem Bereich von 10 mbar bis 100 mbar, insbesondere 10 mbar bis 80 mbar.

In einer Ausführungsform wird als Verdampfer ein Dünnschichtverdampfer verwendet, bei denen die Ausgangs-Lösung im Verdampfer als Flüssigkeitsfilm vorliegt.

Besonders bevorzugt sind vertikale Dünnschichtverdampfer; derartige vertikale Dünnschichtverdampfer sind unter Gerätebezeichnungen wie "Luwa" oder insbesondere "Sambay" der Firmen Buss oder Sulzer bekannt.

Der Dünnschichtverdampfer kann ohne oder mit rotierendem Wischblatt eingesetzt werden.

Bei den bevorzugten vertikalen Dünnschichtverdampfern handelt es sich letztlich um ein senkrechtes Rohr mit innenliegenden Vorrichtungen zur Verteilung und Durchmischung der Ausgangs-Lösung und außenliegenden Vorrichtungen zur Beheizung der Rohrwand.

Die Ausgangs-Lösung wird vorzugsweise im oberen Teil des Dünnschichtverdampfers zugeführt und als Film auf die beheizte Rohrwand verteilt. Der Leichtsieder kann dem Verdampfer, vorzugsweise dem Dünnschichtverdampfer, zusammen mit der Ausgangs-Lösung oder an einer beliebigen anderen Stelle des Verdampfers zugeführt werden. Die Ausgangs-Lösung und der Leichtsieders können in dem Verdampfer in gleicher Richtung (Gleichstrom) oder entgegengesetzt (Gegenstrom) geführt werden.

Vorzugsweise wird der Leichtsieder im Gegenstrom zur Ausgangs-Lösung geführt. Dazu werden die Ausgangs-Lösung insbesondere im oberen Teil des Verdampfers und der Wasserdampf im unteren Teil des Verdampfers zugeführt. Der Leichtsieder und die flüchtigen Bestandteile der Ausgangs-Lösung werden vorzugsweise über einen Abscheider am Kopf des Verdampfers ausgeführt und kondensiert (Destillat).
Die nichtflüchtigen Bestandteile durchlaufen den Verdampfer und werden als flüssiges Sumpfprodukt abgetrennt.

Abbildung 1(FIG 1) zeigt eine entsprechende Apparatur aus Dünnschichtverdampfer (Sambay) und Vorrichtung zur Kondensation.

In einer alternativen Ausführungsform wird zur Umsetzung und Abtrennung eine Destillationskolonne, bevorzugt eine Abtriebskolonne verwendet. Die Abtriebskolonne kann aus einem senkrechten Rohr mit externer Beheizung und mehreren Trennstufen für die Flüssigkeits-(Dampf- Gleichgewichtseinstellung bestehen. Der Zulauf erfolgt vorzugsweise auf den Kopf der Abtriebskolonne.

In einer weiteren alternativen Ausführungsform ist über der Destillationskolonne ein Tropfenabschneider (Demister) angeordnet. Damit wird Tropfenmitriß (ein Zeichen von nicht eingestelltem Gleichgewicht und gleichzeitig fluiddynamischer / strömungstechnischer Überbelastung) verhindert.

Die Ausgangs-Lösung wird vorzugsweise auf den Kopf der Destillationskolonne zugeführt. Der Leichtsieder kann dem Verdampfer zusammen mit der Ausgangs-Lösung oder an einer beliebigen anderen Stelle des Verdampfers zugeführt werden. Die Ausgangs-Lösung und der Leichtsieder können in dem Verdampfer in gleicher Richtung (Gleichstrom) oder entgegengesetzt (Gegenstrom) geführt werden.
Vorzugsweise wird der Leichtsieder im Gegenstrom zur Ausgangs-Lösung geführt. Dazu werden die Ausgangs-Lösung insbesondere im oberen Teil des Verdampfers und der Leichtsieder im unteren Teil des Verdampfers zugeführt.
Der Leichtsieder und die flüchtigen Bestandteile der Ausgangs-Lösung werden vorzugsweise über einen Abscheider am Kopf des Verdampfers ausgeführt und kondensiert (Destillat).
Die nichtflüchtigen Bestandteile durchlaufen den Verdampfer und werden als flüssiges Sumpfprodukt abgetrennt.

Abbildung 2 (FIG 2) zeigt eine entsprechende Apparatur aus Destillationskolonne und Vorrichtung zur Kondensation mit einfachem Zulauf.

Abbildung 3 (FIG 3) zeigt eine entsprechende Apparatur aus Destillationskolonne und Vorrichtung zur Kondensation mit getrennten beheizbaren Zuläufen und Mischer.

Die Reaktion in Verfahrenschritt b) kann je nach Wunsch so durchgeführt werden, dass nur ein Teilumsatz des Saccharids zum HMF oder ein vollständiger Umsatz des Saccharids zu HMF erfolgt. Bei einem Teilumsatz kann nicht umgesetztes Saccharid erneut umgesetzt werden, bei einem vollständigen Umsatz kann es vermehrt zur Bildung von Nebenprodukten, insbesondere sogenannten Huminen, das sind Oligomere des HMF, kommen.

Vorzugsweise werden mindestens 60 %, insbesondere mindestens 80 % und in einer besonderen Ausführungsform mindestens 90 % des eingesetzten Saccharids umgesetzt.

Zu Verfahrensschritt c)

Als Destillat wird eine verdünnte, HMF- enthaltende Lösung erhalten. Das Destillat enthält das bei der Umsetzung entstandene HMF und Wasser sowie den Leichtsieder aus der Destillation. Vorzugsweise enthält das Destillat das bei der Umsetzung entstandene HMF und Wasser aus der Reaktion und Destillation.

Das Destillat enthält insbesondere mehr als 60%, insbesondere mehr als 80% des bei der Umset-zung insgesamt erhaltenen HMF.

Das Destillat enthält insbesondere mindestens 3 Gew.% HMF, besonders bevorzugt mindestens 4 Gew.% HMF und ganz besonders bevorzugt mindestens 6 Gew.% HMF bezogen auf das Ge-samtgewicht des Destillats.

Darüber hinaus kann das Destillat auch Hochsieder enthalten. Im Falle der Verwendung von Polyether oder ionischen Flüssigkeiten als Hochsieder, enthält das Destillat keinen oder nur sehr geringe Mengen an Hochsieder; der Gehalt an Polyether oder ionischen Flüssigkeiten im Destillat ist dann insbesondere kleiner 5 Gew. %, vorzugsweise kleiner 2 Gew. % und besonders be-vorzugt kleiner 1 bzw. kleiner 0,5 Gew. %, bezogen auf das Gesamtgewicht des Destillats.

Nebenprodukte, die bei der Umsetzung des Saccharids zu HMF entstehen sind insbesondere Humine (Oligomere des HMF). Die Humine fallen beim erfindungsgemäßen Verfahren im Wesentlichen nicht im Destillat sondern im Sumpf (siehe Abbildung 1) an.

Das Destillat enthält daher keine oder nur sehr geringe Mengen an Huminen; der Gehalt an Huminen im Destillat ist im Allgemeinen kleiner 2 Gew. %, insbesondere kleiner 0,5 Gew.% und besonders bevorzugt kleiner 0,1 Gew. %. bezogen auf das Gesamtgewicht des Destillats. Das Destillat ist klar und hat eine leichte Gelb- oder Orangefärbung (je nach HMF-Gehalt).

Weiterhin enthält das Destillat keine oder nur geringe Mengen an nicht umgesetztem Saccharid; nicht umgesetztes Saccharid befindet sich überwiegend im Sumpf.

Der Gehalt an nicht umgesetztem Saccharid im Destillat ist im Allgemeinen kleiner 5 Gew. %, insbesondere

kleiner 2 Gew. % und besonders bevorzugt kleiner 1 Gew. % bezogen auf das Gesamtgewicht des Destillats.

Es ist ein Vorteil des erfindungsgemäßen Verfahrens, dass Nebenprodukte der HMF Synthese, Polyether oder ionische Flüssigkeiten als Hochsieder und nicht umgesetztes Saccharid im Wesentlichen im Sumpf anfallen.

HMF wird beim erfindungsgemäßen Herstellungsverfahren direkt als Destillat mit hoher Reinheit erhalten. Das erfindungsgemäße Verfahren ist daher ein einfaches und effektives Verfahren zur Herstellung von HMF und gleichzeitigen Abtrennung von HMF von Nebenprodukten und nicht umgesetzten Ausgangsstoffen.

Das Destillat eignet sich für chemische Synthesen, bei denen HMF als Ausgangsstoff eingesetzt wird. Insbesondere eignet sich das Destillat für chemische Synthesen bei denen der Ausgangs-stoff HMF in hoher Reinheit gewünscht oder benötigt wird. Exemplarisch sei hier die Verwendung der Produkt-Lösung zur Herstellung von 2,5-Furandicarbonsäure oder von 2,5-Bis(hydroxymethyl)furan genannt.

Beispiele

**Beispiel 1: Batchversuche zur HMF-Herstellung in IL mit variablem Wassergehalt**

Die Versuche wurden batchweise durchgeführt in einem Glas-Rundkolben mit Rückflusskühler, mechanischem Rührer und einer Ölbad-Heizung.

Die Ausgangs-Lösungen enthielten: Fructose (20g), Hochsieder (100g), p-Toluolsulfonsäure (1 mol% bez. auf Fructose) und variable Mengen an Wasser

Als Hochsieder wurden verwendet:

PEG-600: ein Polyethylenglycol mit einem Molekulargewicht von 600
BMIMCI: 1-Butyl-3-methylimidazoliumchlorid (BMIM Cl, Basionic ST 70),

Durchführung der Batch-Reaktionen:

Alle Substanzen wurden im Rundkolben vorgelegt, zügig auf 100°C erhitzt und nach Erreichen der Zieltemperatur wurde die Zeitnahme gestartet und in regelmäßigen Abständen Proben aus dem Reaktionsgefäß gezogen zur Reaktionskontrolle.

Die Zusammensetzung wurde mittels HPLC bestimmt.

Die angegebenen Umsätze an Fructose ergeben sich aus den Restmengen an Fructose in den analysierten Proben; Fructose wurde umgesetzt zu HMF und zu Nebenprodukten (Huminen). Die Ausbeute HMF ist der prozentuale Anteil des gebildeten HMF bezogen auf den Fructose-Gehalt in der Ausgangs-Lösung.

Tabelle 1: Batchversuche zur HMF-Herstellung in IL mit variablem Wassergehalt

| Lösemittel | Wassergehalt | Zeit | Umsatz (%) | Ausbeute (%) |
|---|---|---|---|---|
| BMIMCI | Ohne | <10 Min | 93% | 84% |
| BMIMCI | Ca. 10 Gew.% | 1h | 98% | 74% |
| BMIMCI | Ca. 20 Gew.% | 2h | 99% | 74% |
| PEG-600 | Ohne | 8h | 97% | 43% |
| PEG-600 | 2 Gew.% | 8h (18h) | 94% (98%) | 33% (43%) |

**Beispiel 2: in-situ Dehydratisierung von Fructose und Isolation von HMF mittels Wasserdampfdestillation**

Ausgangs-Lösung

Die Ausgangs-Lösungen wurden erhalten durch Mischen von Reinsubstanzen.
Die Ausgangs-Lösungen enthielten Fructose, Hochsieder, Säure und Wasser (siehe Tabelle).

Als Hochsieder wurden verwendet:

DMSO: Dimethylsulfoxid
PEG-600: ein Polyethylenglycol mit einem Molekulargewicht von 600
Tetraglyme: Tetraethylenglycoldimethylether

Als Säuren wurden verwendet:

- $H_2SO_4$: Schwefelsäure
- p-TSA: para-Toluolsulfonsäure
- MSA: Methansulfonsäure
- Oxalsäure

Durchführung der Wasserdampfdestillation

Die Wasserdampfdestillation wurde in der Apparatur gemäß Abbildung 1 durchgeführt. Die Apparatur besteht aus einem Glassambay, der in Gegenstromfahrweise betrieben wird.

Die Ausgangs-Lösung wurde am Kopf zugeführt und mittels eines Überströmers ins Vakuum geflasht, der Wasserdampf als Leichtsieder im unteren Drittel.

Zusammensetzung der Ausgangs-Lösung für verschiedene Hochsieder sowie die gewählten Temperaturen und Drücke sind in der Tabelle aufgeführt.

Die angegebene Temperatur ist diejenige des Heizmediums an der Rohraußenwand, welche in guter Näherung derjenigen des flüssigen Film der Ausgangs-Lösung an der Rohrinnenwand entspricht.

Die Versuche wurden kontinuierlich durchgeführt, nach jeder neuen Temperatur- und Druckeinstellung wurde gewartet bis ein stationärer Zustand erreicht war.

Die Zusammensetzung wurde mittels HPLC bestimmt.

Die angegebenen Umsätze an Fructose ergeben sich aus den Restmengen an Fructose im Sumpf und Destillat; Fructose wurde umgesetzt zu HMF und zu Nebenprodukten (Huminen). Die angegebenen katalytischen Mengen an Säure sind bezogen auf Fructose. Die Ausbeute HMF ist der molare prozentuale Anteil des HMF im Destillat bzw. im Sumpf, bezogen auf den Fructose-Gehalt in der Ausgangs-Lösung.

$$\text{Ausbeute HMF: } \frac{m(HMF)/M/HMF)}{m(Fru)/M\ (Fru)} *100$$

Tabelle 2: kontinuierliche Versuche zur in-situ Dehydratisierung von Fructose und Isolation von HMF

| Hochsieder | Konz. Hochsieder [Gew.%] | Konz. Fructose [Gew.%] | Säure | Menge Säure [mol%, bezogen auf mol Fructose] | Temperatur [°C] | Druck [mbar] | Fructose [Gew.%] im | | HMF [Gew.%] im | | Umsatz an Fructose [%] | Ausbeute HMF [%] | |
|---|---|---|---|---|---|---|---|---|---|---|---|---|---|
| | | | | | | | Destillat | Sumpf | Destillat | Sumpf | | Destillat | Sumpf |
| Tetraglyme | 50 | 10 | Oxalsäure | 18 | 160 | 180 | 0,01 | 0,00 | 0,44 | 0,0 | 99,7 | 18,6 | 0,0 |
| DMSO[1] | 90 | 10 | $H_2SO_4$ | 0,9 | 160 | 180 | 0,00 | 0,00 | 0,90 | 0,00 | 100,0 | 17,21 | 0,01 |
| DMSO | 90 | 10 | $H_2SO_4$ | 0,01 | 110 | 380 | 0,00 | 5,43 | 0,05 | 2,94 | 72,1 | 1,0 | 21,6 |
| PEG-600 | 49 | 24 | MSA | 0,6 | 160 | 25 | 0,17 | 5,73 | 1,73 | 0,22 | 80,9 | 19,1 | 1,0 |
| PEG-600 | 49 | 24 | MSA | 1,0 | 160 | 30 | 0,16 | 0,13 | 1,87 | 0,26 | 98,5 | 19,9 | 1,0 |
| PEG-600 | 49 | 24 | p-TSA | 1,0 | 160 | 30 | 0,03 | 0,07 | 2,12 | 0,39 | 99,6 | 22,2 | 0 |
| PEG-600 | 49 | 24 | Oxalsäure | 1,0 | 160 | 30 | 0,17 | 12,72 | 0,40 | 0,31 | 65,7 | 4,0 | 1,2 |

[1] mit DMSO als zugesetztem Lösemittel wurde unter diesen Temperatur und Druckeinstellungen kein Sumpf erhalten, die gesamte Menge an HMF, DMSO und Wasser findet sich im Destillat.

**Beispiel 3: Isolierung von HMF aus HMF/Hochsiederlösungen mit unterschiedlichen Leichtsiedem**

Ausgangs-Lösung

Die Ausgangs-Lösungen wurden erhalten durch Mischen von Reinsubstanzen.
Die Ausgangs-Lösungen enthielten HMF (10 Gew.%), Fructose (10 Gew.%), Wasser (20 Gew.%) und PEG-600 (60 Gew.%) bezogen auf das Gesamtgewicht der Ausgangslösung. In dieser Lösung findet keine Umsetzung von Fructose zu HMF statt, da keine Säure zugegeben wurde.
Die Fructose wurde lediglich zugegeben, um zu zeigen dass diese lediglich zu sehr geringem Anteil mit ins Destillat übergeht.

Durchführung der Destillation

Die Destillation wurde in der Apparatur gemäß Abbildung 1 durchgeführt. Die Apparatur besteht aus einem Glassambay, der in Gegenstromfahrweise betrieben wird.
Die Ausgangs-Lösung wurde am Kopf zugeführt und mittels eines Überströmers ins Vakuum geflasht, der Leichtsieder im unteren Drittel. Der Leichtsieder wird mittels elektrischer Heizung auf 125°C erhitzt und durch einen Überströmer entspannt und in die Destillationskolonne eingeführt.
Die angegebene Temperatur ist diejenige des Heizmediums an der Rohraußenwand, welche in guter Näherung derjenigen des flüssigen Film der Ausgangs-Lösung an der Rohrinnenwand entspricht.
Die Versuche wurden kontinuierlich durchgeführt, nach jeder neuen Temperatur- und Druckeinstellung wurde gewartet bis ein stationärer Zustand erreicht war.
Die Zusammensetzung wurde mittels HPLC bestimmt.
Die Ausbeute HMF ist der prozentuale Anteil des HMF im Destillat bzw. im Sumpf, bezogen auf den HMF-Gehalt in der Ausgangs-Lösung.
Die Destillationstrennleistung berechnet sich aus prozentualer Anteil HMF im Destillat / prozentualer Anteil HMF im Sumpf *100
Die Fructose Wiederfindung berechnet sich aus prozentualer Anteil Fructose im Sumpf/ prozentualer Anteil Fructose in der Ausgangslösung *100 und soll zeigen, dass unter diesen Bedingungen Fructose nicht umgesetzt wird und zum größten Teil im Sumpf wiedererhalten wird. Tabelle 3: Ergebnisse der Isolierung von HMF aus HMF/Hochsiederlösungen mit unterschiedlichen Leichtsiedern

| Hochsieder | Leichtsieder | Ausbeute HMF (%) | | Fructose-Gehalt im Destillat (Gew.%) | Destillations-Trennleistung HMF Destillat/Sumpf |
|---|---|---|---|---|---|
| | | im Destillat | im Sumpf | | |
| PEG-600 | $H_2O$, | 82,3 | 7,6 | 0,06 | 91,5 |
| PEG-600 | ohne Leichtsieder, | 12,1 | 82,7 | 0,12 | 12,8 |
| PEG-600 | 2-BuOH, | 43,2 | 49,7 | 0,07 | 46,5 |
| PEG-600 | EGDME, | 29,5 | 53,9 | 0,09 | 35,4 |
| PEG-600 | MiBK, | 51,2 | 54,5 | 0,23 | 48,4 |
| PEG-600 | Bu-Ac, | 18,1 | 66,9 | 0,16 | 21,3 |
| PEG-600 | Toluol, | 31,6 | 57,78 | 0,15 | 35,4 |

**Beispiel 4: in-situ Dehydratisierung von Fructose und Isolation von HMF in einer Abtriebskolonne**

Ausgangs-Lösung

Die Ausgangs-Lösungen wurden erhalten durch Mischen von Reinsubstanzen.
Die Ausgangs-Lösungen enthielten Fructose (20 Gew.%), Hochsieder (s. Tabelle), Säure (1 mol% bez. auf Fructose) und Wasser (20 Gew.%) bezogen auf das Gesamtgewicht der Ausgangslösung.
Als Hochsieder wurden verwendet:

PEG-600: ein Polyethylenglycol mit einem Molekulargewicht von 600
EMImCl: 1-Ethyl-3-methylimidazoliumchlorid (EMIM Cl, Basionic ST 80)

Als Säure wurde para-Toluolsulfonsäure verwendet.

Durchführung der Reaktivdestillation

Die Reaktivdestillation wurde in der Apparatur gemäß Abbildung 2 durchgeführt. Die Apparatur besteht aus einer zweiteiligen thermostatierten Glas-Vigreuxkolonne mit einem Reaktionsteil (unten) und einem Demister (oberhalb des Zulaufs der Ausgangslösung). Die Apparatur wird in Gegenstromfahrweise betrieben und die Ausgangslösung wird über einen einzelnen Zulauf zugeführt und mittels eines Überströmers ins Vakuum geflasht.

Die Ausgangs-Lösung wurde zwischen Demister und Reaktionsteil zugeführt, der Leichtsieder im unteren Drittel der Kolonne.

Zusammensetzung der Ausgangs-Lösung für verschiedene Hochsieder sowie die gewählten Temperaturen und Drücke sind in der Tabelle aufgeführt.

Die angegebene Temperatur ist diejenige des Heizmediums an der Kolonnenaußenwand, welche in guter Näherung derjenigen der Flüssigkeit der Ausgangs-Lösung an der Kolonneninnenwand entspricht.
Die Versuche wurden kontinuierlich durchgeführt, nach jeder neuen Temperatur- und Druckeinstellung wurde gewartet bis ein stationärer Zustand erreicht war.

Die Zusammensetzung wurde mittels HPLC bestimmt.

**Die angegebenen Umsätze an Fructose ergeben sich aus den Restmengen an Fructose im Sumpf und Destillat; Fructose wurde umgesetzt zu HMF und zu Nebenprodukten (Huminen).**
Die angegebenen katalytischen Mengen an Säure sind bezogen auf Fructose. Die Ausbeute HMF ist der prozentuale Anteil des HMF im Destillat bzw. im Sumpf, bezogen auf den Fructose-Gehalt in der Ausgangs-Lösung.

Die Destillationstrennleistung berechnet sich aus prozentualer Anteil HMF im Destillat / prozentualer Anteil HMF im Sumpf *100

Tabelle 4: kontinuierliche Versuche zur in-situ Dehydratisierung von Fructose und Isolation von HMF

| Eintrag | Hochsieder | Fluss Ausgangslösung (g/min) | Temp. Destillation (°C) | Druck Destillation (mbar) | Fluss Leichtsieder (g/min) | Leichtsieder | Verhältnis Leichtsieder/ Ausgangslösung | Konz. HMF Destillat (Gew.%) | Konz. Fructose Destillat (Gew.%) | Konz. HMF Sumpf (Gew.%) | Konz. Fructose Sumpf (Gew.%) |
|---|---|---|---|---|---|---|---|---|---|---|---|
| 1 | EMImCl | 2,1 | 140 | 27 | 2,3 | $H_2O$ | 1,1 | 0,72 | 0,00 | 0,67 | 19,10 |
| 2 | EMImCl | 2,1 | 160 | 33 | 2,3 | $H_2O$ | 1,1 | 1,53 | 0,00 | 1,61 | 0 |
| 3 | EMImCl | 2,1 | 180 | 32 | 2,3 | $H_2O$ | 1,1 | 2,88 | 0,00 | 4,09 | 4,09 |
| 4 | EMImCl | 2,4 | 200 | 34 | 2,4 | $H_2O$ | 1,0 | 6,05 | 0,00 | 2,95 | 0,00 |
| 5 | EMImCl | 2,6 | 220 | 35 | 2,5 | $H_2O$ | 1,0 | 8,10 | 0,00 | 1,14 | 0,00 |
| 6 | EMImCl | 2,9 | 180 | 41 | 5,9 | $H_2O$ | 2,0 | 1,53 | 0,00 | 1,06 | 4,72 |
| 7 | EMImCl | 2,7 | 220 | 81 | 2,5 | $H_2O$ | 0,9 | 8,33 | 0,00 | 1,94 | 0,00 |
| **Eintrag** | **Hochsieder** | **Fluss Ausgangslösung (g/min)** | **Temp. Destillation (°C)** | **Druck Destillation (mbar)** | **Fluss Leichtsieder (g/min)** | **Leichtsieder** | **Verhältnis Leichtsieder/ Ausgangslösung** | **Konz. HMF Destillat (Gew.%)** | **Konz. Fructose Destillat (Gew.%)** | **Konz. HMF Sumpf (Gew.%)** | **Konz. Fructose Sumpf (Gew.%)** |
| 8 | EMImCl | 2,8 | 220 | 35 | 1,5 | 2-Butanol | 0,6 | 7,72 | 0,00 | 4,13 | 0,00 |
| 9 | EMImCl | 1,3 | 180 | 28 | 1,0 | $H_2O$ | 1,0 | 4,35 | 0,00 | 5,52 | 2,26 |
| 10 | EMImCl | 1,2 | 180 | 27 | 3,0 | $H_2O$ | 2,5 | 2,79 | 0,00 | 1,56 | 0,87 |
| 11 | EMImCl | 2,5 | 180 | 44 | 2,3 | Methanol | 0,9 | 2,39 | 0,00 | 9,49 | 5,22 |
| 12 | EMImCl | 2,6 | 220 | 45 | 2,3 | Methanol | 0,9 | 7,80 | 0,00 | 1,80 | 0,13 |
| 13 | PEG-600 | 1,2 | 140 | 27 | 2,5 | $H_2O$ | 2,1 | 0,66 | 0,00 | 0,84 | 3,51 |
| 14 | PEG-600 | 1,1 | 160 | 27 | 2,6 | $H_2O$ | 2,3 | 1,54 | 0,00 | 0,89 | 0,83 |
| 15 | PEG-600 | 1,3 | 180 | 29 | 2,5 | $H_2O$ | 2,0 | 2,44 | 0,01 | 0,63 | 0,29 |
| 16 | PEG-600 | 1,1 | 200 | 34 | 2,4 | $H_2O$ | 2,3 | 2,55 | 0,01 | 0,13 | 0,18 |
| 17 | PEG-600 | 1,2 | 220 | 35 | 2,7 | $H_2O$ | 2,2 | 2,60 | 0,03 | 0,03 | 0,02 |

| Eintrag | Gesamt Umsatz Fructose (%) | Ausbeute HMF Destillat (%) | Ausbeute HMF Sumpf (%) | Gesamt Selekt. HMF (%) | Destillations-Trennleistung HMF Dest/Sumpf |
|---|---|---|---|---|---|
| 1 | 17,8 | 5,5 | 4,1 | 53,9 | 57,1 |
| 2 | 100,0 | 12,1 | 8,8 | 20,9 | 57,8 |
| 3 | 86,1 | 25,3 | 19,9 | 52,5 | 56,0 |
| 4 | 100,0 | 50,6 | 13,4 | 64,1 | 79,1 |
| 5 | 100,0 | 62,6 | 5,5 | 68,1 | 92,0 |
| **Ein trag** | **Gesamt Umsatz Fructose (%)** | **Ausbeute HMF Destillat (%)** | **Ausbeute HMF Sumpf (%)** | **Gesamt Selekt. HMF (%)** | **Destillations-Trennleistung HMF Dest/Sumpf** |
| 6 | 67,8 | 16,8 | 10,3 | 40,0 | 62,0 |
| 7 | 100,0 | 62,9 | 11,7 | 74,6 | 84,3 |
| 8 | 100,0 | 51,3 | 16,1 | 67,4 | 76,1 |
| 9 | 92,8 | 36,0 | 24,9 | 65,6 | 59,1 |
| 10 | 94,5 | 46,9 | 14,0 | 64,4 | 77,1 |
| 11 | 82,6 | 19,4 | 45,0 | 78,0 | 30,1 |
| 12 | 99,7 | 58,1 | 6,8 | 65,1 | 89,5 |
| 13 | 81,5 | 10,1 | 6,3 | 20,2 | 61,7 |
| 14 | 96,3 | 25,9 | 5,7 | 32,8 | 81,9 |
| 15 | 98,8 | 38,2 | 3,4 | 42,1 | 91,8 |
| 16 | 99,2 | 44,0 | 0,8 | 45,2 | 98,2 |
| 17 | 99,6 | 42,6 | 0,2 | 42,9 | 99,6 |

Erklärungen:

Eintrag 1-5: Variation der Temperatur von 140°C-220°C mit EMIMCl bei sonst gleichbleibenden Bedingungen. Die Destillationstrennleistung bleibt bis 180°C konstant bei ca. 50% und steigt dann rapide bis auf 92% bei 220°C. Damit lässt sich gut zeigen, dass bei ILs wie EMIMCl das Verfahrensoptimum bei 220°C liegt.

Eintrag 3&6 und 9&10: Erhöhung der Dampfmenge bei sonst gleichbleibenden Bedingungen. Zeigt eine leichte Verbesserung der Destillationsleistung.

Eintrag 5&7: Variation des Vakuums. Bei schlechterem Vakuum von 30 nach 80 mbar sinkt die Destillationsleistung um 8%

Eintrag 5,8,12: Verwendung unterschiedlicher Leichtsieder. Man erhält die gleiche Gesamtselektivität der Reaktion bzgl. HMF aber es zeigen sich deutliche Unterschiede in der Destillationsleistung in der Reihenfolge H2O>MeOH>2-BuOH

Einträge 13-17: Verwendung von PEG-600 bei unterschiedlichen Temperaturen. Das Optimum liegt bei 200°C, dort hat man die höchste HMF-Selektivität bei hoher Destillationsleistung. Allerdings kann man bei den hohen Temperaturen einen leichten Mitriss von Zucker in das Destillat beobachten, der bei ILs nicht vorkommt.

**Beispiel 5: in-situ Dehydratisierung von Fructose und Isolation von HMF in einer Abtriebskolonne mit getrennten Zuläufen.**

Ausgangs-Lösungen

Die Ausgangs-Lösungen wurden erhalten durch Mischen von Reinsubstanzen.
Zulauf 1 ist die Saccharid-Lösung und enthält Fructose (40-70 Gew.%) und Wasser.
Zulauf 2 ist die Hochsieder-Lösung und enthält den Hochsieder (EMIMCl - 95 Gew.%), para-Toluolsulfonsäure (0,44

Gew. %) und Wasser (4,5 Gew.%) bezogen auf das Gesamtgewicht der Ausgangslösung.
Zulauf 3 ist der Leichtsieder (Wasser, Methanol - MeOH oder Ethylacetat - EtOAc).

Durchführung der Reaktivdestillation

Die Reaktivdestillation wurde in der Apparatur gemäß Abbildung 3 durchgeführt. Die Apparatur besteht aus einer zweiteiligen thermostatierten Glas-Vigreuxkolonne mit einem Reaktionsteil (unten) und einem Demister (oberhalb des Zulaufs der Ausgangslösung). Die Apparatur wird in Gegenstromfahrweise betrieben und die Ausgangslösung wird durch Mischen von evtl. vorgeheizten Zulauf 1 und Zulauf 2 erhalten und wird mittels eines Überströmers ins Vakuum geflasht.

Die Ausgangs-Lösung wurde zwischen Demister und Reaktionsteil zugeführt, der Leichtsieder im unteren Drittel der Kolonne.

Die angegebene Temperatur ist diejenige des Heizmediums an der Kolonnenaußenwand, welche in guter Näherung derjenigen der Flüssigkeit der Ausgangs-Lösung an der Kolonneninnenwand entspricht.

Die Versuche wurden kontinuierlich durchgeführt, nach jeder neuen Temperatur- und Druckeinstellung wurde gewartet bis ein stationärer Zustand erreicht war.

Die Zusammensetzung wurde mittels HPLC bestimmt.

**Die angegebenen Umsätze an Fructose ergeben sich aus den Restmengen an Fructose im Sumpf und Destillat; Fructose wurde umgesetzt zu HMF und zu Nebenprodukten (Huminen).** Die angegebenen katalytischen Mengen an Säure sind bezogen auf Fructose. Die Ausbeute HMF ist der prozentuale Anteil des HMF im Destillat bzw. im Sumpf, bezogen auf den Fructose-Gehalt in der Ausgangs-Lösung.

Die Destillationstrennleistung berechnet sich aus prozentualer Anteil HMF im Destillat / prozentualer Anteil HMF im Sumpf *100

Tabelle 5: kontinuierliche Versuche zur in-situ Dehydratisierung von Fructose und Isolation von HMF mit getrennten Zuläufen

| Eintrag | Konz.Fructose in Zul.1 (Gew.%) | Temp. Vorheizer (°C) | Fluss Zul.1 (g/min) | Fluss Zul.2 (g/min) | Säure-Menge (mol%) | Temp. Destillation (°C) | Druck Destillation (mbar) | Fluss Zul.3 (g/min) | Leichtsieder | Verhältnis Zul.2/ Zul.1 |
|---|---|---|---|---|---|---|---|---|---|---|
| 1 | 40 | 180 | 1,3 | 1,2 | 0,9 | 200 | 17 | 2,4 | $H_2O$ | 0,9 |
| 2 | 40 | 180 | 1,3 | 1,1 | 0,8 | 220 | 25 | 2,4 | $H_2O$ | 0,8 |
| 3 | 40 | 180 | 2,5 | 2,2 | 0,8 | 220 | 32 | 2,4 | $H_2O$ | 0,9 |
| 4 | 70 | 180 | 1,5 | 1,1 | 0,7 | 220 | 27 | 2,4 | $H_2O$ | 0,8 |
| 5 | 70 | 180 | 1,5 | 1 | 0,8 | 180 | 22 | 2,4 | $H_2O$ | 0,9 |
| 6 | 40 | 25 | 2,5 | 2,3 | 0,8 | 220 | 18 | 2,4 | $H_2O$ | 0,9 |
| 7 | 40 | 180 | 1,3 | 1,4 | 1,0 | 220 | 35 | 1,9 | MeOH | 1,0 |
| 8 | 40 | 180 | 1,3 | 1,2 | 1,0 | 220 | 18 | 2,3 | EtOAc | 1,0 |
| 9[a] | 40 | 180 | 1,3 | 1,4 | 1,0 | 220 | 28 | 2,4 | $H_2O$ | 1,0 |

| Eintrag | Verhältnis Zul.3/Zul. 1&2 | Konz. HMF Destillat (Gew.%) | Konz. Fructose Destillat (Gew.%) | Konz. HMF Sumpf (Gew.%) | Konz. Fructose Sumpf (Gew.%) | Gesamt Umsatz Fructose (%) | Ausbeute HMF Destillat (%) | Ausbeute HMF Sumpf (%) | Gesamt Selekt. HMF (%) | Destillations-Trennleistung HMF Dest/Sumpf |
|---|---|---|---|---|---|---|---|---|---|---|
| 1 | 0,9 | 7,23 | 0 | 1,67 | 0 | 100 | 65,9 | 5,9 | 71,8 | 91,8 |
| 2 | 1 | 7,21 | 0 | 0,66 | 0 | 100 | 69,8 | 2,4 | 72,2 | 96,7 |
| 3 | 0,5 | 10,52 | 0 | 1,05 | 0 | 100 | 70,9 | 3,6 | 74,5 | 95,2 |
| 4 | 0,9 | 10,32 | 0,00 | 2,27 | 0,00 | 100,0 | 51,1 | 4,1 | 55,2 | 92,6 |

| | | | | | | | | | | |
|---|---|---|---|---|---|---|---|---|---|---|
| 5 | 0,9 | 7,9 | 0,0 | 5,68 | 2,91 | 94,9 | 33,8 | 14,3 | 50,8 | 70,2 |
| **Eintrag** | **Verhältnis Zul.3/Zul. 1&2** | **Konz. HMF Destillat (Gew.%)** | **Konz. Fructose Destillat (Gew.%)** | **Konz. HMF Sumpf (Gew.%)** | **Konz. Fructose Sumpf (Gew.%)** | **Gesamt Umsatz Fructose (%)** | **Ausbeute HMF Destillat (%)** | **Ausbeute HMF Sumpf (%)** | **Gesamt Selekt. HMF (%)** | **Destillations-Trennleistung HMF Dest/Sumpf** |
| 6 | 0,5 | 7,41 | 0,00 | 3,88 | 0,62 | 98,2 | 42,8 | 16,1 | 60,0 | 72,6 |
| 7 | 0,7 | 6,58 | 0,00 | 1,01 | 0,00 | 100,0 | 58,5 | 4,3 | 60,6 | 93,0 |
| 8 | 0,9 | 17,89 | 0,00 | 1,76 | 0,00 | 100,0 | 54,1 | 6,8 | 60,9 | 88,8 |
| 9[a] | 0,9 | 7,25 | 0,00 | 0,38 | 0,13 | 99,6 | 69,7 | 1,6 | 71,5 | 97,8 |

[a] in der Hochsieder-Lösung wurde EMIM $CH_3SO_3$ statt EMIM Cl verwendet

Erklärungen:

Eintrag 1&2: Durch die Erhöhung der Reaktions/Destillationstemperatur wird eine bessere Destillations-Trennleistung erreicht, bei gleichbleibender Gesamtselektivität der Reaktion.

Eintrag 2&3: Eine Verringerung des Leichtsieders ist ökonomisch und technisch sinnvoll und liefert bei gleichbleibender Gesamtselektivität sowie Destillationstrennleistung hohe HMF-Gehalte im Destillat von ca. 10 Gew.%.

Eintrag 2&4: Wird eine höhere Konzentration an Fructose im Zulauf 1 verwendet, sinkt der Gesamtwassergehalt im System und man erhält eine schlechtere Selektivität der Reaktion, weil das gebildete HMF nicht schnell genug aus

dem Reaktionsmedium entfernt werden kann

Eintrag 4&5: Ein Verringerung der Reaktionstemperatur auf 180°C führt auch bei hohen Fructose-Gehalten im Zulauf 1 zu einer schlechteren Destillations-Trennleistung und lediglich zu einem Teilumsatz der Fructose.

Eintrag 3&6: Wenn die Zuläufe 1 &2 nicht beheizt werden zeigt sich eine schlechtere Destillations-Trennleistung und eine geringere Gesamtselektivität der Reaktion. Dies liegt wahrscheinlich daran, dass durch eine Erhitzung der wässrigen Saccharid-Lösung das Wasser bereits in einem überkritischen Zustand vorliegt und durch die schlagartige Entspannung in das Vakuum deutlich besser verdampft und somit das gebildete HMF mit sich reißt (besserer Flash)

Eintrag 7&8: Als Leichtsieder können auch Methanol und Ethylacetat verwendet werden aber man erhält leicht schlechtere Destillations-Trennleistungen, sowie Gesamtselektivitäten der Reaktion im Vergleich zu Wasser. Mit Ethylacetat wird ein zweiphasiges Destillat erhalten, bei dem der Hauptanteil des HMF in der wässrigen Phase vorliegt (die angegebene Ausbeute bezieht sich auf das gesamte Destillat).

Eintrag 9: Die Reaktion kann mit gleichbleibenden Ausbeuten auch mit EMIM $CH_3SO_3$ durchgeführt werden.

**Beispiel 6: in-situ Dehydratisierung von Glucose oder Glucose/Fructose Mischungen und Isolation von HMF in einer Abtriebskolonne mit getrennten Zuläufen.**

Ausgangs-Lösungen

Die Ausgangs-Lösungen wurden erhalten durch Mischen von Reinsubstanzen.
Zulauf 1 ist die Saccharid-Lösung und enthält Glucose (40 Gew.%) oder Glucose/Fructose (Verh. 58:42 - 40 Gew.% Gesamtzucker) und Wasser.

Zulauf 2 ist die Hochsieder-Lösung und enthält den Hochsieder (EMIMCl - 92,95 Gew.%), $CrCl_3$ (1,72 Gew.%), para-Toluolsulfonsäure (0,42 Gew. %) und Wasser (4,91 Gew.%) bezogen auf das Gesamtgewicht der Ausgangslösung. Zulauf 3 ist der Leichtsieder (Wasser).

Durchführung der Reaktivdestillation

Die Reaktivdestillation wurde in der Apparatur gemäß Abbildung 3 durchgeführt. Die Apparatur besteht aus einer zweiteiligen thermostatierten Glas-Vigreuxkolonne mit einem Reaktionsteil (unten) und einem Demister (oberhalb des Zulaufs der Ausgangslösung). Die Apparatur wird in Gegenstromfahrweise betrieben und die Ausgangslösung wird durch Mischen von evtl. vorgeheizten Zulauf 1 und Zulauf 2 erhalten und wird mittels eines Überströmers ins Vakuum geflasht.

Die Ausgangs-Lösung wurde zwischen Demister und Reaktionsteil zugeführt, der Leichtsieder im unteren Drittel der Kolonne.

Die angegebene Temperatur ist diejenige des Heizmediums an der Kolonnenaußenwand, welche in guter Näherung derjenigen der Flüssigkeit der Ausgangs-Lösung an der Kolonneninnenwand entspricht.

Die Versuche wurden kontinuierlich durchgeführt, nach jeder neuen Temperatur- und Druckeinstellung wurde gewartet bis ein stationärer Zustand erreicht war.

Die Zusammensetzung wurde mittels HPLC bestimmt.

**Die angegebenen Umsätze der Gesamtzucker ergeben sich aus den Restmengen an Glucose und Fructose im Sumpf und Destillat; Glucose und Fructose wurden umgesetzt zu HMF und zu Nebenprodukten (Huminen).** Die angegebenen katalytischen Mengen an Säure sind bezogen auf Gesamtzucker. Die Ausbeute HMF ist der prozentuale Anteil des HMF im Destillat bzw. im Sumpf, bezogen auf den gesamtzucker-Gehalt in der Ausgangs-Lösung.

Die Destillationstrennleistung berechnet sich aus prozentualer Anteil HMF im Destillat / prozentualer Anteil HMF im Sumpf *100

Tabelle 6: kontinuierliche Versuche zur in-situ Dehydratisierung von Glucose oder Glucose/Fructose Mischungen und Isolation von HMF mit getrennten Zuläufen.

| Ein-trag | Feed | Temp. Vor-heizer (°C) | Fluss Zulauf 1 (g/min) | Fluss Zulauf 2 (g/min) | Säure-Menge (mol%) | Temp. Destillation (°C) | Druck Destillation (mbar) | Fluss Zulauf 3 (g/min) | Leichtsieder |
|---|---|---|---|---|---|---|---|---|---|
| 1 | Glucose | 180 | 1,3 | 1,3 | 0,8 | 220 | 27 | 2,4 | $H_2O$ |
| 2 | Glucose | 180 | 1,3 | 1,3 | 0,8 | 220 | 33 | 1,3 | $H_2O$ |
| 3 | Glucose | 180 | 1,3 | 1,2 | 0,8 | 180 | 37 | 2,2 | $H_2O$ |
| 4 | Glu/Fru (58:42) | 180 | 1,3 | 1,3 | 0,8 | 220 | 37 | 2,3 | $H_2O$ |
| 5 | Glu/Fru (58:42) | 180 | 1,3 | 1,3 | 0,8 | 220 | 35 | 1,3 | $H_2O$ |
| 6[a] | Glucose | 180 | 1,3 | 1,4 | 1,0 | 200 | 22 | 2,4 | $H_2O$ |

| Ein-trag | Verh. Zul.2/Zul.1 | Verh. Zul.3/Zul. 1&2 | Aus-beute HMF Destillat (%) | Aus-beute Zucker Destillat (%) | Aus-beute HMF Sumpf (%) | Aus-beute Zucker Sumpf (%) | Gesamt Umsatz Zucker (%) | Gesamt Selekt. HMF (%) | Gesamt Aus-beute HMF (%) | Destil-lations-Trenn-leistung HMF Dest/Sumpf |
|---|---|---|---|---|---|---|---|---|---|---|
| 1 | 0,9 | 0,9 | 51,8 | 0,0 | 0,4 | 0,4 | 99,6 | 52,4 | 52,2 | 99,2 |
| 2 | 1,0 | 0,5 | 47,6 | 0,0 | 2,7 | 0,6 | 99,4 | 50,6 | 50,3 | 94,4 |
| 3 | 0,9 | 0,9 | 19,7 | 0,0 | 34,3 | 8,9 | 91,1 | 59,3 | 54,0 | 36,9 |
| 4 | 1,0 | 0,9 | 51,7 | 0,0 | 0,5 | 0,7 | 99,3 | 52,5 | 52,2 | 99,0 |
| 5 | 0,9 | 0,5 | 51,8 | 0,0 | 0,6 | 0,6 | 99,4 | 52,6 | 52,3 | 98,9 |
| 6[a] | 1,0 | 0,9 | 54,2 | 0,6 | 2,9 | 0,4 | 99,0 | 57,6 | 57,1 | 95,0 |

[a] in der Hochsieder-Lösung wurde EMIM $CH_3SO_3$ statt EMIM Cl verwendet

Erklärung:

Eintrag 1&2: Die Reaktion kann auch mit Glucose durchgeführt werden und liefert gute Ausbeuten an HMF im Destillat. Im Vergleich zu Fructose (Tabelle 4) ist die Reaktion mit Glucose etwas unselektiver und führt vermehrt zu unerwünschten Nebenreaktionen, wenn das gebildete HMF nicht schnell genug aus der Reaktionslösung abgetrennt werden kann.
Eintrag 3: Bei einer niedrigeren Reaktionstemperatur von 180°C, steigt zwar die Selektivität zum HMF, jedoch sinkt gleichzeitig die Destillations-Trennleistung.
Eintrag 4&5: Die Reaktion mit einer Mischung aus Glucose und Fructose führt zu ähnlich guten Ausbeuten an HMF wie mit reiner Glucose.
Eintrag 6: Die Reaktion kann mit gleichbleibenden Ausbeuten auch mit EMIM $CH_3SO_3$ durchgeführt werden.

## Patentansprüche

**1.** Verfahren zur Herstellung von 5-Hydroxymethylfurfural (HMF), **dadurch gekennzeichnet, dass**

a) wässrige Lösungen (im Nachfolgenden Ausgangs-Lösung genannt), welche

- ein oder mehrere Hexosen und
- ein organisches Lösemittel mit einem Siedepunkt größer 200°C (bei Normaldruck) (kurz Hochsieder genannt) und
- Wasser enthalten

und ein Lösemittel mit einem Siedepunkt größer 60°C und kleiner 200°C (bei Normaldruck, kurz Leichtsieder genannt) einem Reaktionsgefäß zugeführt werden,
b) in dem Reaktionsgefäß eine Umsetzung der Hexose zu HMF in Gegenwart von Wasserdampf bei gleichzeitiger destillativer Abtrennung des HMF erfolgt und
c) als Destillat eine wässrige, HMF- enthaltende Lösung (im Nachfolgenden Destillat genannt) erhalten wird.

**2.** Verfahren gemäß Anspruch 1, **dadurch gekennzeichnet, dass** das Verhältnis der zugeführten Menge an Leichtsieder zur Menge der zugeführten Ausgangs-Lösung in einem Bereich von 0,2 bis 4 Gewichtseinheiten Leichtsieder auf 1 Gewichtseinheit Ausgangs-Lösung liegt.

**3.** Verfahren gemäß Anspruch 1 oder 2, **dadurch gekennzeichnet, dass** es sich bei der ein oder mehreren Hexose um Fructose, Glucose oder Gemische von Fructose und Glucose handelt.

**4.** Verfahren gemäß einem der vorigen Ansprüche, **dadurch gekennzeichnet, dass** es sich bei dem Hochsieder um einen Polyether oder eine ionische Flüssigkeit handelt

**5.** Verfahren gemäß einem der vorigen Ansprüche, **dadurch gekennzeichnet, dass** es sich bei der ionischen Flüssigkeit um Imidazoliumchloride oder Imidazoliummethansulfonate, besonders bevorzugt um 1-Ethyl-3-methylimidazoliumchlorid (EMIM Cl), 1-Butyl-3-methylimidazoliumchlorid (BMIM Cl), 1-Ethyl-3-methylimidazoliummethansulfonat (EMIM $CH_3SO_3$) oder 1-Butyl-3-methylimidazoliummethansulfonat (BEMIM $CH_3SO_3$) oder Mischungen davon handelt

**6.** Verfahren gemäß einem der vorigen Ansprüche, **dadurch gekennzeichnet, dass** die Ausgangslösung durch folgende Schritte hergestellt wird:

A1) Die ein oder mehrere Hexosen und Wasser liegen in einem Reaktionsgefäß vor.
A2) In einem weiteren Reaktionsgefäß wird der Hochsieder und vorzugsweise ein Katalysator vorgelegt.
A3) Unmittelbar vor dem Verfahrensschritt b) werden die Komponenten gemischt, vorzugsweise in einer Mischkammer,

wobei die Lösungen der Schritte A1 und A2 vorzugsweise in den beiden Reaktionsgefäßen getrennt voneinander vor dem Mischen auf eine Temperatur zwischen 150 und 200°C vorgeheizt werden,

**7.** Verfahren gemäß einem der vorigen Ansprüche, **dadurch gekennzeichnet, dass** der Leichtsieder Wasserdampf,

Methanol, oder 2-Butanol ist.

8. Verfahren gemäß einem der vorigen Ansprüche, **dadurch gekennzeichnet, dass** die Ausgangs-Lösung den Hochsieder in Mengen von 5 bis 90 Gew. % enthält.

9. Verfahren gemäß einem der vorigen Ansprüche, **dadurch gekennzeichnet, dass** die Ausgangslösung ein Metallchlorid oder Metallnitrat der allgemeinen Formel MXn enthält, wobei M ein Metall, X Chlor oder Nitrat und n eine ganze Zahl von 1 bis 4 ist.

10. Verfahren gemäß einem der Ansprüche 1 bis 9, **dadurch gekennzeichnet, dass** die Umsetzung zu HMF in Gegenwart einer in der Ausgangs-Lösung löslichen Säure erfolgt.

11. Verfahren gemäß einem der vorigen Ansprüche, **dadurch gekennzeichnet, dass** die Umsetzung zu HMF bei 100°C bis 250°C erfolgt.

12. Verfahren gemäß einem der vorigen Ansprüche, **dadurch gekennzeichnet, dass** die Umsetzung zu HMF bei einem Druck von 10 bis 200 mbar erfolgt.

13. Verfahren gemäß einem der vorigen Ansprüche, **dadurch gekennzeichnet, dass** das Verfahren kontinuierlich durchgeführt wird, wobei die Ausgangslösung und der Leichtsieder dem Verdampfer kontinuierlich zugeführt werden und die Produkt-Lösung kontinuierlich abgeführt wird.

14. Verfahren gemäß einem der vorigen Ansprüche, **dadurch gekennzeichnet, dass** es sich bei dem Reaktionsgefäß um einen Dünnschichtverdampfer oder eine Abtriebskolonne handelt.

15. Verfahren gemäß einem der vorigen Ansprüche, **dadurch gekennzeichnet, dass** der Leichtsieder im Gegenstrom zur Ausgangs-Lösung zugeführt wird.

16. Verfahren gemäß einem der vorigen Ansprüche, **dadurch gekennzeichnet, dass** sich mehr als 60% des erhaltenen HMF im Destillat befinden.

17. Verfahren gemäß einem der vorigen Ansprüche, **dadurch gekennzeichnet, dass** das Destillat zur Herstellung von 2,5-Furandicarbonsäure oder 2,5-Bis(hydroxymethyl)furan verwendet wird.

18. Verfahren gemäß einem der vorigen Ansprüche, **dadurch gekennzeichnet, dass** das Verfahren in grosstechnischem Maßstab durchgeführt wird.

19. Verfahren gemäß einem der vorigen Ansprüche, **dadurch gekennzeichnet, dass** die Ausgangs-Lösung mit dem Leichtsieder im Reaktionsgefäß in Kontakt gebracht wird.

## Claims

1. A process for preparing 5-hydroxymethylfurfural (HMF), which comprises

   a) feeding aqueous solutions (hereinafter called starting solution) comprising

      - one or more hexoses and
      - an organic solvent having a boiling point greater than 200°C (at standard pressure) (called high boiler for short) and
      - water,

   and a solvent having a boiling point greater than 60 °C and less than 200°C (at standard pressure, called low boiler for short) to a reaction vessel,
   b) effecting a conversion of hexose to HMF in the reaction vessel in the presence of water vapor with simultaneous distillative removal of the HMF and
   c) obtaining, as the distillate, an aqueous, HMF-comprising solution (hereinafter called distillate).

**2.** The process according to claim 1, wherein the ratio of the amount of low boiler supplied to the amount of the starting solution supplied is within a range from 0.2 to 4 weight units of low boiler to 1 weight unit of starting solution.

**3.** The process according to claim 1 or 2, wherein the one or more hexoses are fructose, glucose or mixtures of fructose and glucose.

**4.** The process according to any of the preceding claims, wherein the high boiler is a polyether or an ionic liquid.

**5.** The process according to any of the preceding claims, wherein the ionic liquid comprises imidazolium chlorides or imidazolium methanesulfonates, more preferably 1-ethyl-3-methylimidazolium chloride (EMIM Cl), 1-butyl-3-methylimidazolium chloride (BMIM Cl), 1-ethyl-3-methylimidazolium methanesulfonate (EMIM $CH_3SO_3$) or 1-butyl-3-methylimidazolium methanesulfonate (BEMIM $CH_3SO_3$) or mixtures thereof.

**6.** The process according to any of the preceding claims, wherein the starting solution is prepared by the following steps:

A1) the one or more hexoses and water are present in a reaction vessel,
A2) a further reaction vessel is initially charged with the high boiler and preferably a catalyst,
A3) immediately prior to process step b), the components are mixed, preferably in a mixing chamber,

the solutions of steps A1 and A2 preferably being preheated to a temperature between 150 and 200°C in the two reaction vessels separated from one another prior to the mixing.

**7.** The process according to any of the preceding claims, wherein the low boiler is water vapor, methanol or 2-butanol.

**8.** The process according to any of the preceding claims, wherein the starting solution comprises the high boiler in amounts of 5 to 90% by weight.

**9.** The process according to any of the preceding claims, wherein the starting solution comprises a metal chloride or metal nitrate of the general formula MXn where M is a metal, X is chlorine or nitrate and n is an integer from 1 to 4.

**10.** The process according to any of claims 1 to 9, wherein the conversion to HMF is effected in the presence of an acid soluble in the starting solution.

**11.** The process according to any of the preceding claims, wherein the conversion to HMF is effected at 100°C to 250°C.

**12.** The process according to any of the preceding claims, wherein the conversion to HMF is effected at a pressure of 10 to 200 mbar.

**13.** The process according to any of the preceding claims, which is performed continuously, the starting solution and the low boiler being supplied continuously to the evaporator and the product solution being removed continuously.

**14.** The process according to any of the preceding claims, wherein the reaction vessel is a thin-film evaporator or a stripping column.

**15.** The process according to any of the preceding claims, wherein the low boiler is supplied in countercurrent to the starting solution.

**16.** The process according to any of the preceding claims, wherein more than 60% of the HMF obtained is present in the distillate.

**17.** The process according to any of the preceding claims, wherein the distillate is used for preparation of 2,5-furandicarboxylic acid or 2,5-bis(hydroxymethyl)furan.

**18.** The process according to any of the preceding claims, which is conducted on the industrial scale.

**19.** The process according to any of the preceding claims, wherein the starting solution is contacted with the low boiler in the reaction vessel.

## Revendications

1. **1.** Procédé de fabrication de 5-hydroxyméthylfurfural (HMF), **caractérisé en ce que**

   a) des solutions aqueuses (par la suite nommées solution initiale), qui contiennent

   - un ou plusieurs hexoses et
   - un solvant organique ayant un point d'ébullition supérieur à 200 °C (à pression normale) (en abrégé nommé composant de point d'ébullition élevé) et
   - de l'eau,

   et un solvant ayant un point d'ébullition supérieur à 60 °C et inférieur à 200 °C (à pression normale, en abrégé nommé composant de point d'ébullition faible) sont introduits dans un récipient de réaction,
   b) une réaction de l'hexose en HMF a lieu dans le récipient de réaction en présence de vapeur d'eau avec séparation par distillation simultanée de l'HMF, et
   c) une solution aqueuse contenant de l'HMF (par la suite nommée distillat) est obtenue en tant que distillat.

**2.** Procédé selon la revendication 1, **caractérisé en ce que** le rapport entre la quantité de composant de point d'ébullition faible introduite et la quantité de solution initiale introduite se situe dans une plage allant de 0,2 à 4 unités en poids de composant de point d'ébullition faible sur 1 unité en poids de solution initiale.

**3.** Procédé selon la revendication 1 ou 2, **caractérisé en ce que** le ou les hexoses sont le fructose, le glucose ou des mélanges de fructose et de glucose.

**4.** Procédé selon l'une quelconque des revendications précédentes, **caractérisé en ce que** le composant de point d'ébullition élevé est un polyéther ou un liquide ionique.

**5.** Procédé selon l'une quelconque des revendications précédentes, **caractérisé en ce que** le liquide ionique consiste en des chlorures d'imidazolium ou des méthane-sulfonates d'imidazolium, de manière particulièrement préférée le chlorure de 1-éthyl-3-méthylimidazolium (EMIM Cl), le chlorure de 1-butyl-3-méthylimidazolium (BMIM Cl), le méthane-sulfonate de 1-éthyl-3-méthylimidazolium (EMIM $CH_3SO_3$) ou le méthane-sulfonate de 1-butyl-3-méthylimidazolium (BEMIM $CH_3SO_3$) ou leurs mélanges.

**6.** Procédé selon l'une quelconque des revendications précédentes, **caractérisé en ce que** la solution initiale est fabriquée par les étapes suivantes :

   A1) le ou les hexoses et de l'eau sont chargés dans un récipient de réaction,
   A2) le composant de point d'ébullition élevé et de préférence un catalyseur sont chargés dans un autre récipient de réaction,
   A3) les composants sont mélangés immédiatement avant l'étape de procédé b), de préférence dans une chambre de mélange,

   les solutions des étapes A1 et A2 étant de préférence préchauffées dans les deux récipients de réaction séparément l'une de l'autre avant le mélange à une température comprise entre 150 et 200 °C.

**7.** Procédé selon l'une quelconque des revendications précédentes, **caractérisé en ce que** le composant de point d'ébullition faible est la vapeur d'eau, le méthanol ou le 2-butanol.

**8.** Procédé selon l'une quelconque des revendications précédentes, **caractérisé en ce que** la solution initiale contient le composant de point d'ébullition élevé en quantités de 5 à 90 % en poids.

**9.** Procédé selon l'une quelconque des revendications précédentes, **caractérisé en ce que** la solution initiale contient un chlorure de métal ou un nitrate de métal de formule générale MXn, M étant un métal, X le chlore ou un nitrate et n un nombre entier de 1 à 4.

**10.** Procédé selon l'une quelconque des revendications 1 à 9, **caractérisé en ce que** la réaction en HMF a lieu en présence d'un acide soluble dans la solution initiale.

11. Procédé selon l'une quelconque des revendications précédentes, **caractérisé en ce que** la réaction en HMF a lieu à une température de 100 °C à 250 °C.

12. Procédé selon l'une quelconque des revendications précédentes, **caractérisé en ce que** la réaction en HMF a lieu à une pression de 10 à 200 mbar.

13. Procédé selon l'une quelconque des revendications précédentes, **caractérisé en ce que** le procédé est réalisé en continu, la solution initiale et le composant de point d'ébullition faible étant introduits en continu dans l'évaporateur et la solution de produit étant déchargée en continu.

14. Procédé selon l'une quelconque des revendications précédentes, **caractérisé en ce que** le récipient de réaction est un évaporateur à couche mince ou une colonne de rectification.

15. Procédé selon l'une quelconque des revendications précédentes, **caractérisé en ce que** le composant de point d'ébullition faible est introduit à contre-courant de la solution initiale.

16. Procédé selon l'une quelconque des revendications précédentes, **caractérisé en ce que** plus de 60 % de l'HMF obtenu se trouve dans le distillat.

17. Procédé selon l'une quelconque des revendications précédentes, **caractérisé en ce que** le distillat est utilisé pour la fabrication d'acide 2,5-furanedicarboxylique ou de 2,5-bis(hydroxyméthyl)furane.

18. Procédé selon l'une quelconque des revendications précédentes, **caractérisé en ce que** le procédé est réalisé à l'échelle industrielle.

19. Procédé selon l'une quelconque des revendications précédentes, **caractérisé en ce que** la solution initiale est mise en contact avec le composant de point d'ébullition faible dans le récipient de réaction.

FIG 1:

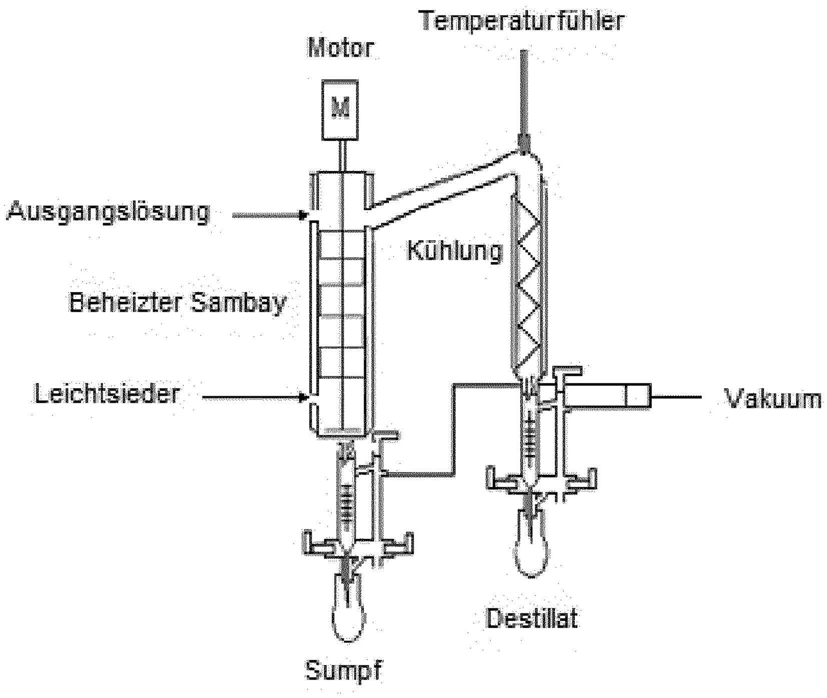
Temperaturfühler
Motor
M
Ausgangslösung
Kühlung
Beheizter Sambay
Leichtsieder
Vakuum
Destillat
Sumpf

**FIG 2:**

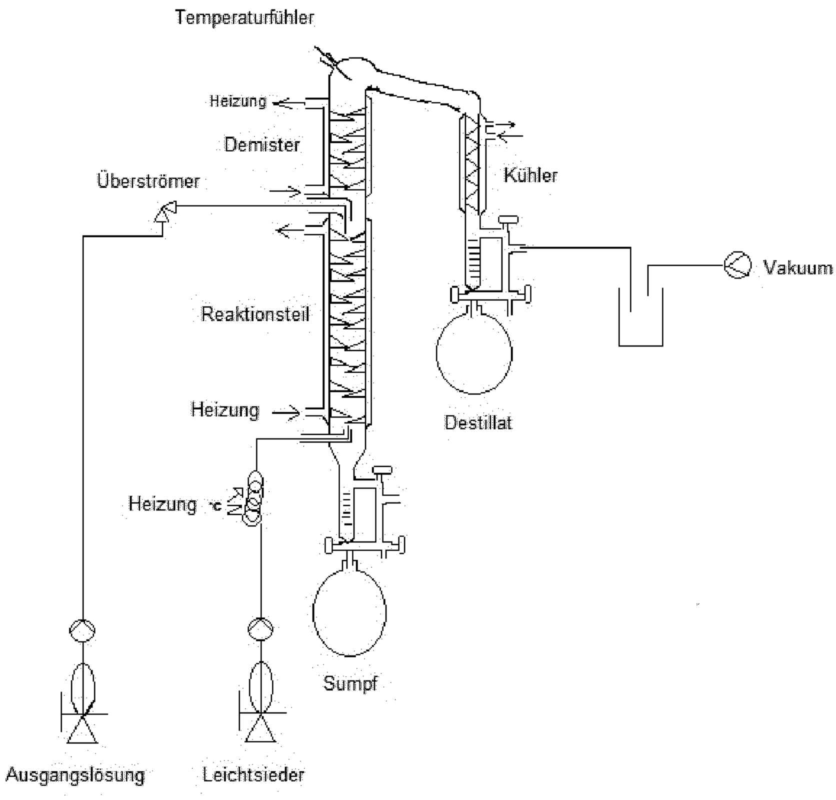
Temperaturfühler
Heizung
Demister
Überströmer
Kühler
Vakuum
Reaktionsteil
Destillat
Heizung
Heizung °c
Sumpf
Ausgangslösung
Leichtsieder

FIG 3:

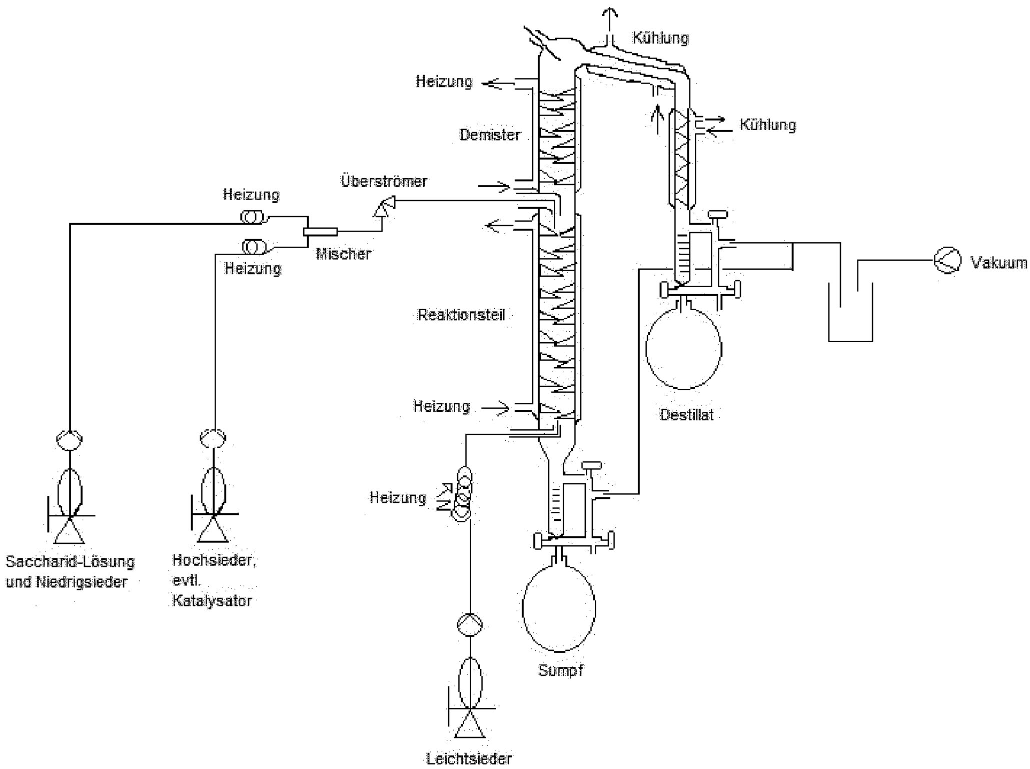
Kühlung
Heizung
Demister
Kühlung
Überströmer
Heizung
Mischer
Heizung
Vakuum
Reaktionsteil
Heizung
Destillat
Heizung
Saccharid-Lösung
und Niedrigsieder
Hochsieder,
evtl.
Katalysator
Sumpf
Leichtsieder

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3601281 A
- EP 1834950 A
- EP 1834951 A
- EP 2033958 A1
- FR 2663933
- FR 2664273
- US 4400468 A
- CN 102399203
- WO 2008090155 A
- WO 2008090156 A
- DE 10202838 A1


### In der Beschreibung aufgeführte Nicht-Patentliteratur


- **FEROZ KABIR KAZI et al.** *Chem Eng.,* 2011, vol. 169, 329-338
- **MARK MASCAL ; EDWARD B. NIKITIN.** *Angew. Chemie,* 2008, vol. 47, 7924-7926
- **HARU KAWAMOTO ; SHINYA SAITO et al.** *J. Wood Sci.,* 2007, vol. 53, 127-133
- **WEI et al.** *Green Chem.,* 2012, vol. 14, 1220-1226
- Glucose-isomerization with Chromium-salts. *Science,* 2007, vol. 316, 1597-1600
- *Angew. Chem. Int. Ed.,* 2008, vol. 47, 9345-9348
- *Chem. Eur. J.,* 2011, vol. 17, 5281-5288
- Glucose-isomerization with rare-earth metals. *J. Mol. Cat. A,* 2012, vol. 356, 158-164
- HMF from Glucose with lanthanides. *Green Chem.,* 2010, vol. 12, 321-325
- Conversion of Cellulose to Furans with metal salts. *J. Mol. Catal. A,* 2012, vol. 357, 11-18
- Sn-Beta Zeolithes. *ACS Catal.,* 2011, vol. 1, 408-410
- *Biores. Tech.,* 2011, vol. 102, 4179-4183
- *Carbohydr. Res.,* 1977, vol. 54, 177-183
- *Science,* 2007, vol. 136, 1597-1600
- *Mechanismus der Reaktion siehe Science,* 2006, vol. 312, 1933-1937
- *Science,* 2007, vol. 316, 1597-1600
- *Carbohydr. Pol.,* 2012, vol. 90, 792-798
- *Green Chem.,* 2009, vol. 11, 1746-1749
- **K. N. MARSH et al.** *Fluid Phase Equilibria,* 2004, vol. 219, 93-98
- **J. G. HUDDLESTON et al.** *Green Chemistry,* 2001, vol. 3, 156-164